# EUROPEAN PATENT APPLICATION

(11) **EP 2 168 613 A1**
(43) Date of publication of application: **31.03.2010**
(21) Application number: 08764098.3
(22) Date of filing: 12.06.2008
(51) Int. Cl.: A61M 1/34, A61M 1/14

(54) **BLOOD PURIFICATION APPARATUS AND METHOD OF CONFIRMING CIRCUIT CONTINUITY FAILURE THEREOF**

(30) Priority: 13.06.2007 JP 2007156639
(71) Applicant: Kuraray Medical Inc., Kurashiki-shi Okayama 7100801 (JP)
(72) Inventor: INOUE, Masao, Okayama 7048191 (JP); IKE, Akihiro, Tokyo 100-0004 (JP)
(74) Representative: Jenkins, Peter David
(86) International application number: PCT/JP2008/001503
(87) International publication number: WO 2008/152810

(57) **Abstract**

A blood processing apparatus in which whether or not circuit components are connected is ascertained, and a method of ascertaining a failure in such circuit connection is provided. By closing a blood return valve, disposed in a blood return passage for returning a blood, processed in a first blood processing unit, to patient's body, blocking a blood circuit including a blood introducing passage, the blood return passage and a delivery passage for delivering unnecessary blood component from the first blood processing unit, driving a first pump in the delivery passage in normal direction, and driving an air pump, fluidly connected with the blood introducing passage and the blood return passage, in reverse direction to discharge air from the circuit to generate negative pressure inside the circuit and the first blood processing unit, connection between the circuit and the first blood processing unit is ascertained.

## Description

### CROSS REFERENCE TO THE RELATED APPLICATION

This application is based on and claims Convention priority to Japanese patent application No. 2007-156639, filed June 13, 2007, the entire disclosure of which is herein incorporated by reference as a part of this application.

### BACKGROUND OF THE INVENTION

### (Field of the Invention)

The present invention relates to a blood purification apparatus provided with a blood processing device for separating a blood into a useful blood component and a malefic blood component and a method of ascertaining the presence of a fault in circuit connection in such blood purification apparatus.

### (Description of the Related Art)

As an apparatus used in the medical treatment of some diseases such as, for example, kidney failure, liver failure and autoimmune disease, a slow hemodiafiltration apparatus and a double filtration blood purification apparatus have been well known in the art. The slow hemodiafiltration apparatus includes a dialytic filtering device, into which blood and dialyzing fluid are introduced so that fluid, metabolic waste product and electrolyte can be removed through a diffusion shell within the dialyzing and filtering device based on the difference in concentration between the blood and the dialyzing fluid and the difference in intermembrane pressure. On the other hand, the double filtration blood purification apparatus includes a blood component separator for separating blood into a blood cell component and a plasma component and a plasma component separator for separating the separated plasma component into a high molecular weight component, containing toxins, and a low molecular weight component.

In those types of the blood processing apparatuses, prior to the blood processing or the priming procedure being initiated, it is of prime importance to ascertain whether or not air lines having disposed therein a blood processing device such as, for example, a dialyzing and filtering unit and/or a blood component separator and pressure sensors used to detect pressures within fluid circuits are properly connected with such fluid circuits employed in the blood processing apparatus. Since those types of the blood processing apparatuses make use of the fluid circuits of a complicated circuit configuration and, also, since the circuit configuration of those fluid circuits are often changed depending on the purpose of the blood processing, not only assemblage of the fluid circuits, but also a work to ascertain the presence or absence of failure in circuit connection require skills and, therefore, the apparatuses are considered having a poor usability.

For this reason, demands have been made to provide a method of ascertaining the presence of a failure in circuit connection, which can be accomplished with no skill required. The Patent Document 1 listed below suggests such a connection failure ascertaining method as including driving in a normal direction a plasma pump for introducing the separated plasma component into a plasma adsorber, developing a negative pressure within a portion of a fluid passage upstream of the plasma pump, developing a positive pressure within a portion of the fluid passage downstream of the plasma pump, measuring the positive pressure to determine if an upstream side membrane filter provided in that portion of the fluid passage upstream of the plasma pump, is properly connected, and measuring the negative pressure developed within that portion of the fluid passage to determine if a downstream side membrane filter provided in a portion of the fluid passage downstream of the plasma pump is properly connected.

The Patent Document 2 listed below also suggests such a connection failure ascertaining method as including supplying air from an air pump into a blood introducing passage and a blood delivery passage through a first pressure measuring line, which is fluidly connected with the blood introducing passage for introducing therethrough a blood into a blood processing unit, and a second pressure measuring line, which is fluidly connected with the blood delivery passage for returning the processed blood back to a patient, respectively, so that a positive pressure can be developed inside the blood introducing passage and the blood delivery passage, and measuring the respective positive pressures, developed inside the blood introducing passage and the blood delivery passage, to determine if the blood introducing tube, the blood delivery tube, pressure measuring lines and the blood processing units are properly connected with each other.
[Patent Document 1] JP Laid-open Patent Publication No. H02-289259
[Patent Document 2] JP Laid-open Patent Publication No. 2002-95741

### SUMMARY OF THE INVENTION

It has, however, been found that the connection failure ascertaining method disclosed in the Patent Document 1 discussed above is incapable of ascertaining a failure occurring in fluid connection of the separator (blood component separator) and the absorber, though capable of ascertaining a failure occurring in fluid connection of the membrane filter. On the other hand, the connection failure ascertaining method disclosed in the Patent Document 2 discussed above has such a problem that in the event that although a waste plasma transport passage for discharging therethrough the separated plasma is fluidly connected with a plasma discharge port of the blood processing unit, the attendant worker fails to remove a protective cap from the plasma discharge port when the blood processing unit is installed, the air will be blocked off by the protective cap despite of the positive pressure applied by the air pump and, therefore, no pressure leakage will not occur within the blood processing unit, wherefore it will be erroneously ascertained that the waste plasma transport tube is not properly connected with the blood processing unit.

In view of the foregoing, the present invention has been devised to substantially eliminate the above discussed problems and inconveniences inherent in the prior art and has for its primary object to provide a blood processing apparatus designed to enable it to be ascertained whether or not circuit components such as, for example, the blood processing unit and pressure air lines are properly connected, and a method of ascertaining the presence of a failure in such circuit connection.

In order to accomplish these objects, the present invention provides a blood processing apparatus which includes a blood circuit, including a first blood processing unit for separating a blood into a first blood component, containing a useful subcomponent, and a second blood component containing a harmful subcomponent, a blood introducing passage for introducing therethrough the blood into the blood processing unit, a blood return passage for returning the separated first blood component back to a patient's body, a delivery passage for delivering the separated second blood component from the first blood processing unit; a first pump disposed in the delivery passage for delivering fluid; a blood return valve disposed in the blood return passage; an air pump fluidly connected with the blood introducing passage and also with the blood return passage; and a controller for controlling the first pump, the air pump and the blood return valve.

The controller forming a part of the blood processing apparatus of the present invention in turn includes a blood side connection ascertaining mode setting section for generating a negative pressure inside the blood circuit and also inside the first blood processing unit by causing the blood return valve to be closed and the blood circuit to be blocked and, also, causing the first pump and the air pump to be driven in a normal direction and a reverse direction, respectively, to discharge air from the blood circuit. The controller referred to above also includes a blood side connection ascertaining section for ascertaining, when the negative pressure attains a first predetermined pressure value within a predetermined time, a fluid connection between the blood circuit and the first blood processing unit.

According to another aspect of the present invention, there is provided a method of ascertaining the presence of a failure in circuit connection, which is executed by a blood purification apparatus designed to separate a blood into a first blood component, containing a useful subcomponent, and a second blood component containing a harmful subcomponent, introduce the blood into a first blood processing unit through a blood introducing passage, return the separated first blood component back to a patient's body through a blood return passage, and deliver the separated second blood component from the first blood processing unit through a delivery passage. This connection failure ascertaining method of the present invention includes closing a blood return valve, disposed in the blood return passage, to block a blood circuit including the blood introducing passage, the blood return passage and the delivery passage; driving the first pump, disposed in the delivery passage, in a normal direction, and driving an air pump, fluidly connected with the blood introducing passage and also with the blood return passage, in a reverse direction to cause air to be discharged from the blood circuit to allow a negative pressure to be developed inside the blood passage and also inside the first blood processing unit; and ascertaining that, when the negative pressure attains a first predetermined pressure value within a predetermined time, the blood circuit and the first blood processing unit are fluidly connected with each other.

According to the above described apparatus of the present invention and the above described method of the present invention, when the air pump fluid connected commonly with the blood introducing passage, through which the blood is introduced into the first blood processing unit, and the blood return passage through which the blood is returned back to the patient's body, in the reverse direction and the first pump is driven in the normal direction, the negative pressure can be generated within the blood circuit, including the blood introducing passage, the blood return passage and the delivery passage, and the first blood processing unit and, therefore, if as a result of measurement of the negative pressure, such negative pressure attains the first predetermined pressure value within the predetermined time, it can be ascertained that the various passages are properly fluidly connected with the first blood processing unit with no pressure leakage occurring. Also, in addition to the capability of ascertaining the circuit connection, it can also be ascertained if the circuit is blocked by, for example, a clamp or forceps.

In a preferred embodiment of the present invention, the blood purification apparatus may be a blood filtering and dialyzing apparatus having a second pump installed and equipped with a dialysis liquid introducing passage for introducing a dialysis liquid into the first blood processing unit. In this case, when the second pump is driven in a normal direction to allow air to flow into the first blood processing unit, a positive pressure is generated within the first blood processing unit and, accordingly, it can be ascertained that the dialysis liquid introducing passage and the first blood processing unit are fluidly connected with each other in the event that the positive pressure referred to above attains a second predetermined pressure value within a predetermined time.

According to this preferred embodiment, since the positive pressure can be generated within the first blood processing unit when as a result of the drive of the second pump in the normal direction air is introduced from the dialysis liquid introducing passage into the first blood processing unit, measurement of this positive pressure enables the leakage-free connection between the dialysis liquid introducing passage and the first blood processing unit to be ascertained in the event that the measured positive pressure attains the second predetermined pressure value within the predetermined time. This makes it possible to ascertain if the dialyzing fluid introducing passage is properly connected with the first blood processing unit, when the attendant worker fails to remove, for example, a protective cap from a connection port of the first blood processing unit, at which the dialysis liquid introducing passage is to be connected, at the time of, for example, installation of the first blood processing unit onto the apparatus.

In another preferred embodiment of the present invention, the blood purification apparatus may be formed as a double filtration blood purification apparatus equipped with a second blood processing unit fluidly connected with the delivery passage for separating the second blood component into a high molecular weight subcomponent and a low molecular weight subcomponent. In this case, when the first pump is driven in a reverse direction to allow air to flow into the first blood processing unit, a positive pressure is generated within the first blood processing unit and, accordingly, it can be ascertained that the delivery passage and the first blood processing unit are fluidly connected with each other in the event that the positive pressure referred to above attains a second predetermined pressure value within a predetermined time.

According to this preferred embodiment, since the positive pressure can be generated within the first blood processing unit when as a result of the drive of the first pump in the normal direction air is introduced from the delivery passage into the first blood processing unit, measurement of this positive pressure enables the leakage-free connection between the delivery passage and the first blood processing unit to be ascertained in the event that the measured positive pressure attains the second predetermined pressure value within the predetermined time. This design makes it possible to ascertain if the delivery passage is properly connected with the first blood processing unit, when the attendant worker fails to remove, for example, a protective cap from a connection port of the first blood processing unit, at which the dialysis liquid introducing passage is to be connected, at the time of, for example, installation of the first blood processing unit onto the apparatus.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a schematic diagram showing a slow hemodiafiltration apparatus, which is a blood purification apparatus according to a first preferred embodiment of the present invention;
Fig. 2 is a fluid circuit diagram showing the manner of ascertaining the presence of a failure in circuit connection in the slow hemodiafiltration apparatus shown in Fig. 1;
Fig. 3 is a fluid circuit diagram showing the manner of ascertaining the presence of a failure in circuit connection in the slow hemodiafiltration apparatus shown in Fig. 1;
Fig. 4 is a fluid circuit diagram showing the manner of ascertaining the presence of a failure in circuit connection in the slow hemodiafiltration apparatus shown in Fig. 1;
Fig. 5 is a fluid circuit diagram showing the manner of ascertaining the presence of a failure in circuit connection in the slow hemodiafiltration apparatus shown in Fig. 1;
Fig. 6 is a fluid circuit diagram showing the manner of ascertaining the presence of a failure in circuit connection in the slow hemodiafiltration apparatus shown in Fig. 1;
Fig. 7 is a fluid circuit diagram showing the manner of ascertaining the presence of a failure in circuit connection in the slow hemodiafiltration apparatus shown in Fig. 1;
Fig. 8 is a schematic diagram showing a double filtration blood purification apparatus according to a second preferred embodiment of the present invention;
Fig. 9 is a fluid circuit diagram showing the manner of ascertaining the presence of a failure in circuit connection in the blood purification apparatus shown in Fig. 8;
Fig. 10 is a fluid circuit diagram showing the manner of ascertaining the presence of a failure in circuit connection in the blood purification apparatus shown in Fig. 8;
Fig. 11 is a fluid circuit diagram showing the manner of ascertaining the presence of a failure in circuit connection in the blood purification apparatus shown in Fig. 8;
Fig. 12 is a fluid circuit diagram showing the manner of ascertaining the presence of a failure in circuit connection in the blood purification apparatus shown in Fig. 8;
Fig. 13 is a fluid circuit diagram showing the manner of ascertaining the presence of a failure in circuit connection in the blood purification apparatus shown in Fig. 8;
Fig. 14 is a fluid circuit diagram showing the manner of ascertaining the presence of a failure in circuit connection in the blood purification apparatus shown in Fig. 8;
Fig. 15 is a fluid circuit diagram showing the manner of ascertaining the presence of a failure in circuit connection in the blood purification apparatus shown in Fig. 8;
Fig. 16 is a fluid circuit diagram showing the manner of ascertaining the presence of a failure in circuit connection in the blood purification apparatus shown in Fig. 8;
Fig. 17 is a fluid circuit diagram showing the manner of ascertaining the presence of a failure in circuit connection in the blood purification apparatus shown in Fig. 8; and
Fig. 18 is a fluid circuit diagram showing the manner of ascertaining the presence of a failure in circuit connection in the blood purification apparatus shown in Fig. 8.

### DETAILED DESCRIPTION OF THE EMBODIMENTS

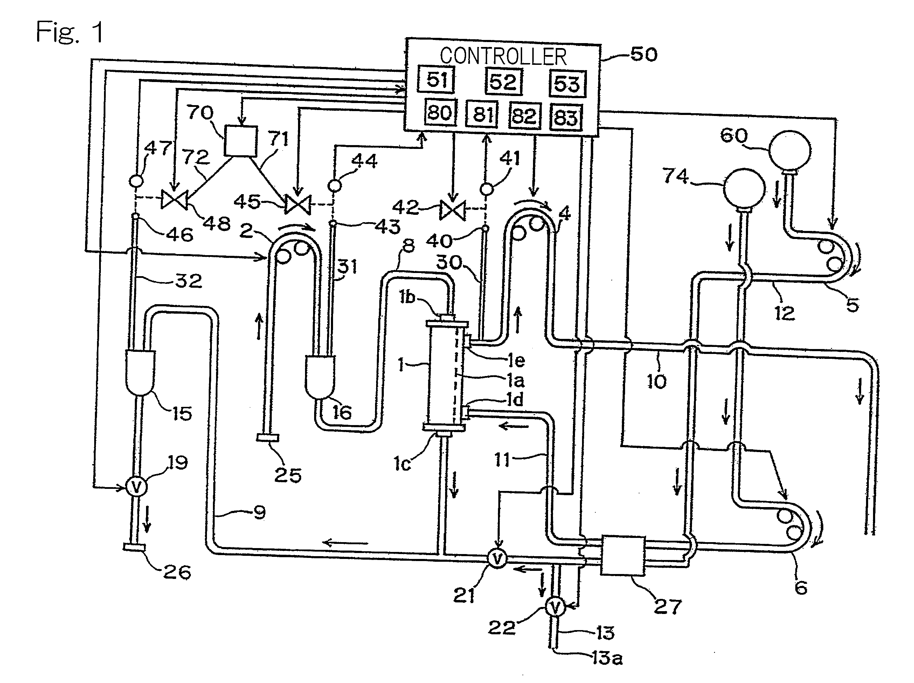

Hereinafter, preferred embodiments of the present invention will be described in detail with reference to the accompanying drawings. At the outset, a slow hemodiafiltration apparatus, in which a method of ascertaining the presence of a failure in circuit connection according to the embodiment of the present invention is performed, will be described. In particular, Fig. 1 illustrates a schematic diagram showing a slow hemodiafiltration apparatus, which is a blood purification apparatus according to a first preferred embodiment of the present invention. The slow blood filtering and dialyzing apparatus shown therein includes a dialyzing and filtering unit forming a first blood processing unit 1. This first blood processing unit 1 is of a type capable of removing, for example, water, metabolic decomposition products and/or electrolyte from a blood by the utilization of the concentration difference and the intermembrane pressure difference between the blood and a dialysis liquid, introduced into the dialyzing and filtering unit, which are subsequently discharged as a filtrate, in which they are mixed with the dialysis liquid, to thereby purify the blood.

The first blood processing unit 1 is in the form of, for example, a cylindrical housing having a hollow fiber or flat plate, tubular separating membrane (filtering membrane) 1a accommodated therein. The separating membrane 1a of the blood processing unit 1 has a multiplicity of pores defined therein and having a pore size within the range of 0.002 to 0.01 µm and is employed in the form of a homogeneous microporous membrane, a microfiltration membrane or an asymmetric membrane made up of a porous support layer and a microporous structural layer. Although as a membrane 1a for the blood processing unit 1 various separating membranes have been well known, the use is preferred of the separating membrane, having an excellent biocompatibility, made of a copolymer of an ethylene vinyl alcohol (EVA) system, a cellulose derivative, a PMMA membrane, or polysulfone.

The first blood processing unit 1 is fluidly connected with a blood introducing passage 8 for introducing therethrough a blood, drawn from a blood introducing element 25 (an element that can be communicated with an ordinary blood collecting vessel such as, for example, a shunt and a syringe needle, or a blood reservoir), into the first blood processing unit 1. This blood introducing passage 8 is in turn fluidly connected with a blood introducing pump 2 and then with a blood drip chamber 16, both disposed in an upstream portion of the blood introducing passage 8 with respect to the direction of flow of the blood. The blood introduced from the blood introducing element 25 into the blood introducing passage 8 is, after the pressure thereof has been increased by the blood introducing pump 2, supplied into the blood drip chamber 16. Subsequently the blood so supplied into the blood drip chamber 16 is supplied dropwise from the blood drip chamber 16 into the first blood processing unit 1 by way of a blood inlet 1b, defined in an upper (upstream) end portion thereof, so that the blood so introduced can be separated by the separating membrane 1a into water, metabolic waste products and electrolyte to thereby purify the blood.

The first blood processing unit 1 is also fluidly connected with a blood return passage 9 through which the purified blood, which is a first blood component, can be returned to a patient's body. This blood return passage 9 is provided with, from an upstream side thereof, a return blood drip chamber 15 and then with a blood return valve 19. The blood so purified emerges outwardly from the first blood processing unit 1 by way of a blood outlet 1c defined in a lower (downstream) end portion thereof and is then supplied into the return blood drip chamber 15. Thereafter, the purified blood is dropwise supplied from the return blood drip chamber 15 and is, after having been introduced to a blood delivery element 26 (an element that can be communicated with a shunt or an intravenous drip kit) by way of a blood return valve 19 then opened, returned to the patient's body.

The first blood processing unit 1 has a side surface and a portion of this side surface of the first blood processing unit 1 proximate to the blood inlet 1b is provided with a first connection port 1e. This first connection port 1e is a filtrate discharge port through which a filtrate, which is a second blood component and contains, for example, water, metabolic products and electrolyte, can be discharged from the first blood processing unit 1. This first connection port 1e is fluidly connected with a delivery passage 10 for delivering (discharging) the filtrate, and a first pump 4 for the delivery of the filtrate is provided in the filtrate delivery passage 10. On the other hand, another portion of the side surface of the first blood processing unit 1 proximate to the blood outlet 1c is provided with a second connection port 1d, which is a dialysis liquid inlet through which a dialysis liquid inflows, and a dialysis liquid introducing passage 11, for introducing the dialysis liquid into the first blood processing unit 1, is fluidly connected with the second connection port 1d of the first blood processing unit 1. This dialysis liquid introducing passage 11 is provided with a dialysis liquid supply source 74, a second pump 6 for introducing the dialysis liquid and a heater 27 arranged in this order from an upstream side. The dialysis liquid emerging from the dialysis liquid supply source 74 is, after the pressure thereof has been increased by the second pump 6, introduced through the heater 27 into the first blood processing unit 1 by way of the second connection port 1d of the first blood processing unit 1. The dialysis liquid so introduced flows along the separating membrane 1a, that is, flows outside the separating membrane 1a (a right side of the separating membrane 1a shown in Fig. 1), subsequently transports water, metabolic waste products and electrolyte, which have filtered by the separating membrane 1a, based on the difference in concentration with the dialysis liquid and the intermembrane pressure difference, and have drawn out of the separating membrane, and is thereafter discharged from the filtrate discharge port 1e into the delivery passage 10. The filtrate so flowing into the delivery passage 10 is finally discharged to the outside of the apparatus after the pressure thereof has been increased by the first pump 4. The blood introducing passage 8, the blood return passage 9 and the delivery passage 10 cooperatively form a blood circuit.

Also, a liquid replacement introducing passage 12 for supplying a liquid replacement to the purified blood emerging from the blood outlet 1c is fluidly connected with the blood return passage 9 at a location generally intermediate between the first connection port 1c of the first blood processing unit 1 and the return blood drip chamber 15. This liquid replacement passage 12 is provided with a third pump 5 for introducing the liquid replacement, a heater 27 and a liquid replacement introducing valve 21 in this order from an upstream side. The liquid replacement emerging from the liquid replacement supply source 60 is, after the pressure thereof has been increased by the third pump 5, introduced into the blood return passage 9 through the heater 27 and then through the liquid replacement introducing valve 21, finally merging with the purified blood. The purified blood and the liquid replacement, which are merged together in the manner described above, subsequently flow into the return blood drip chamber 15 and is then supplied to the patient's body from the blood delivery element 26 by way of the blood return valve 19. A cleansing liquid discharge passage 13 for discharging a cleansing liquid used during the priming procedure to the outside of the apparatus is fluidly connected with the liquid replacement introducing passage 12 at a location generally intermediate between the heater 27 and the liquid replacement valve 21, and this liquid cleansing discharge passage 13 is provided with a cleansing liquid discharge valve 22. During the priming procedure taking place, the used cleansing liquid is discharged to the outside of the apparatus through a cleansing liquid discharge port 13a by way of the cleansing liquid discharge valve 22 then held in an opened condition.

A first air line 30 is fluidly connected with the delivery passage 10 at a location generally intermediate between the first connection port 1e of the first blood processing unit 1 and the first pump 4, and this air line 30 is provided with a first membrane filter 40, a first pressure sensor 41 for detecting the pressure inside the delivery passage 10 and the pressure acting on the first membrane filter 40, and a first air valve 42 for selectively establishing or interrupting the communication of air with the outside of the apparatus.

The blood drip chamber 16 is fluidly connected with a second air line 31, which is provided with a second membrane filter 43, a second pressure sensor 44 for detecting the pressure acting on the second membrane filter 43 and the pressure inside the blood introducing passage 8 through an air pooled within the blood drip chamber 16, and a second air valve 45 for selectively establishing or interrupting the communication of air with the outside of the apparatus. On the other hand, the return blood drip chamber 15 is fluidly connected with a third air line 32, which is provided with a third membrane filter 46, a third pressure sensor 47 for detecting not only the pressure inside the blood return passage 9 through an air pooled inside the return blood drip chamber 15, but also the pressure acting on the third membrane filter 46, and a third air valve 48 for selectively establishing or interrupting the communication of air with the outside of the apparatus. The second air valve 45 referred to above is fluidly connected with an air pump 70 through an air tube 71, and the third air valve 48 referred to above is also fluidly connected with the air pump 70 through an air tube 72. This air pump 70 is of a type capable of introducing the air into the passages through the second air valve 45 and the third air valve 48 and discharging the air from the passages.

The slow blood filtering and dialyzing apparatus of the construction described hereinabove is provided with a controller 50, which has a pump drive unit 51 for driving the blood introducing pump 2, the first pump 4, the second pump 6, the third pump 5 and the air pump 70, a circuit valve drive unit 52 for selectively opening or closing the blood return valve 19, a blood merging valve 21 and the cleansing liquid discharge valve 22, and an air valve drive unit 53 for selectively opening or closing the first air valve 42, the second air valve 45 and the third air valve 48, all built therein. This controller 50 controls the pump drive unit 51 and the valve drive unit 52, based on respective detected pressure signals fed from the first pressure sensor 41, the second pressure sensor 44 and the third pressure sensor 47.

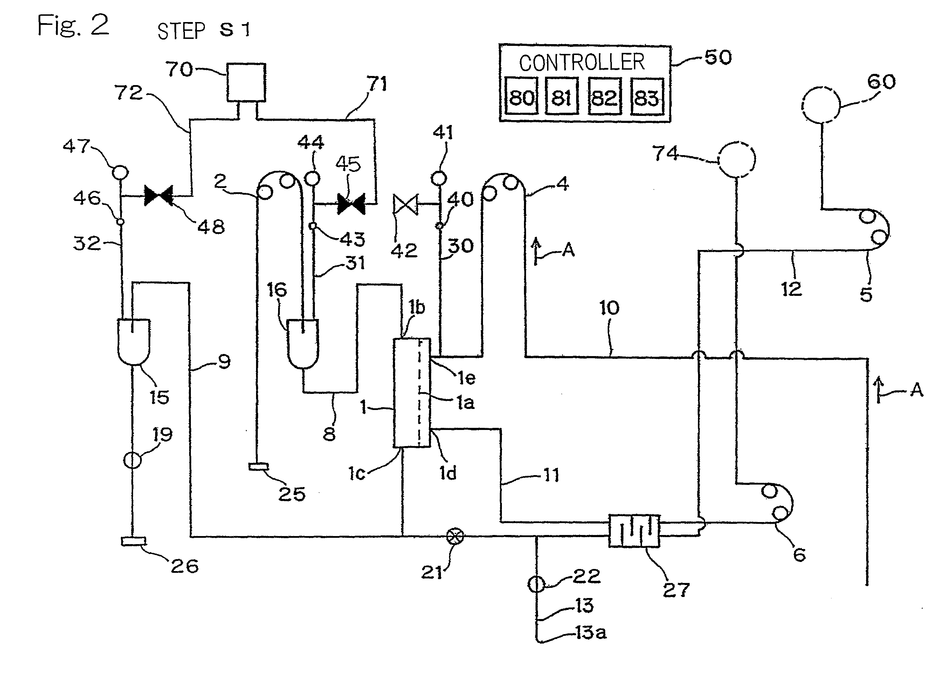

The controller 50 also has a blood side connection ascertaining mode setting section 80, a blood side connection ascertaining section 81, an introduction side connection ascertaining mode setting section 82 and an introduction side connection ascertaining section 83. When the presence of a failure in circuit connection of the slow hemodiafiltration apparatus of the type discussed hereinbefore is to be ascertained, the blood side connection ascertaining mode setting section 80 is operable to control not only the selective opening or closure of the return blood valve 19, but also the rotation of each of the first pump 4 and the air pump 70 to generate a negative pressure inside the air lines 30 and 31 and the first blood processing unit 1. On the other hand, the blood side connection ascertaining section 81 is operable in response to the signal from the first pressure sensor 41 to ascertain that the blood passage and the first blood processing unit 1 are fluidly connected with each other at the time the negative attains a first predetermined pressure value within a predetermined time, and may be constituted by, for example, an alarm and/or a display lamp. At each of steps S 1 to S6 shown in Fig. 2 to 7, respectively, and as will be described in detail later, in the event of a failure being found present in circuit connection, the attendant worker can be informed of the failure occurring in circuit connection by means of sounds generated by the alarm and/or indicator lamp caused by the display lamp.

Also, the introduction side connection ascertaining mode setting section 82 is operable to control not only the selective opening or closure of the blood merging valve 21 and the cleansing liquid discharge valve 22, but also the rotation of the second pump 6 and the third pump 5 to generate a positive pressure inside the liquid replacement introducing passage 12, the dialysis liquid introducing passage 11 and the first blood processing unit 1. On the other hand, the introduction side connection ascertaining section 83 is operable in response to the signal from the first pressure sensor 41 to ascertain that the dialysis liquid introducing passage 11 and the first blood processing unit 1 are fluidly connected with each other at the time the positive pressure referred to above attains a second predetermined pressure value within a predetermined time, and may be constituted by, for example, an alarm and/or a display lamp. As is the case with the blood side connection ascertaining section 81, at each of steps S1 to S6 shown in Fig. 2 to 7, respectively, and as will be described in detail later, the attendant worker can be informed of the failure, occurring in circuit connection, by means of sounds generated by the alarm and/or indicator lamp caused by the display lamp in the event of the failure being found present in circuit connection.

Hereinafter, the procedure for detecting the presence or absence of a fault in circuit connection in the slow hemodiafiltration apparatus will be described in detail with particular reference to steps S 1 to S6 shown respectively in Figs. 2 to 7. It is to be noted that in each of those steps S1 to S6 shown respectively in Figs. 2 to 7, arrow headed lines indicate the directions of flow of air A within the circuit.

At the outset, at step S1, the controller 50 is activated in response to an initiating signal fed from the outside. By this controller 50, the cleansing liquid discharge valve 22 and the first air valve 42 are temporarily opened, followed by closure of the first air valve 42, the second air valve 45, the third air valve 48, the blood return valve 19, the blood merging valve 21 and the cleansing liquid discharge valve 22. At this time, the air pump 70, the blood introducing pump 2, the first pump 4, the second pump 6 and the third pump 5 have not yet been rotated. Since each of those pumps 2, 4, 5 and 6 is pinched at least one or more locations in the corresponding passages, they function as a shielding member for inhibiting movement of air across the respective pump.

Then, at step S2, when by the controller 50, the second air valve 45 and the third air valve 48 are opened, the blood return valve 19 is closed, the air pump 70 is rotated in a reverse direction, and the first pump 4 is rotated in a normal direction, the air flows in a direction shown by the arrow headed lines and, therefore, a negative pressure is developed in a portion of the blood introducing passage 8 downstream of the blood introducing pump 2, the first blood processing unit 1, a portion of the blood return passage 9 upstream of the blood return valve 19, a portion of the delivery passage 10 upstream of the first pump 4, and inside the first air line 30. As a result, the air A within the fluid circuit is discharged from an air discharge port of the air pump 70 to the outside of the apparatus after having flowed through the second air valve 45 in the second air line 31 and the third air valve 48 in the third air line 32 by way of the air tubes 71 and 72. Also, the air A flowing into the filtrate delivery passage 10 is discharged to the outside of the apparatus through the first pump 4.

At step S2, the air pump 70 and the first pump 4 are rotated for a period of, for example, 30 seconds. When within this period of 30 seconds, not only does the second pressure sensor 44 detects that the pressure (negative pressure) acting on the second membrane filter 43 has attained a first predetermined pressure value, for example, -80 mmHg or lower, and the third pressure sensor 47 detects that the pressure acting on the third membrane filter 46 has attained -80 mmHg or lower, the controller 50 causes the air pump 70 to halt. Also, if within the period of 30 seconds, the first pressure sensor 41 detects that the pressure (negative pressure) acting on the first membrane filter has attained -80 mmHg, the controller 50 causes the first pump 4 to halt. In this way, in the event that the first to third pressure sensors 41, 44 and 47 detect the first predetermined pressure value within the period of 30 seconds, it is ascertained that the first blood processing unit 1 is properly fluidly connected with the blood introducing passage 8, the blood return passage 9 and the delivery passage 10 with no pressure leakage occurring therein. Other than that, it can be also ascertained that the first membrane filter 40 and the first pressure sensor 41 are fluidly connected with the first air line 30, the second membrane filter 43 and the second pressure sensor 44 are fluidly connected with the second air line 31 and the third membrane filter 46 and the third pressure sensor 47 are fluidly connected with the third air line 32.

On the other hand, in the event that any one of the first to third pressure sensors 41, 44 and 47 fails to detect the first predetermined pressure value (for example, -80 mmHg or lower in the illustrated embodiment) within the period of 30 seconds, it is ascertained that the first blood processing unit 1 is not properly fluidly connected with the blood introducing passage 8, the blood return passage 9 and the delivery passage 10. Other than that, it can be also ascertained that the first membrane filter 40 and the first pressure sensor 41 are not fluidly connected with the first air line 30, the second membrane filter 43 and the second pressure sensor 44 are not fluidly connected with the second air line 31 and the third membrane filter 46 and the third pressure sensor 47 are not fluid connected with the third air line 32. Thus, in the event that the failure in circuit connection is so ascertained, after such failure in circuit connection has been removed by the attendant worker, the sequence of steps S1 and S2 has to be performed again to ascertain whether or not the circuit connection is properly established.

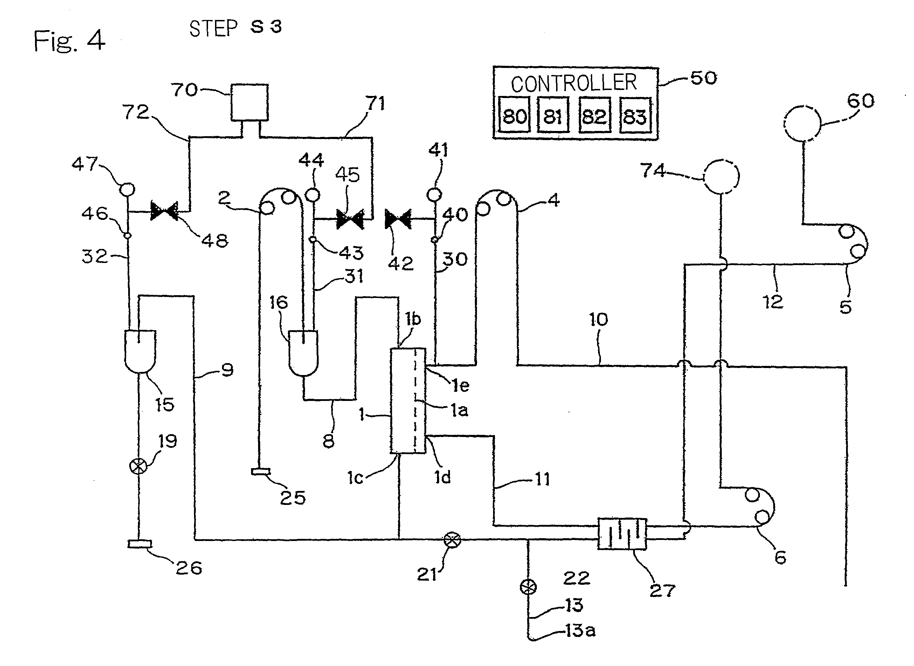

Subsequently, at step S3 shown in Fig. 4, by the controller 50, the second and third air valves 45 and 48 are closed so that all of the air valves are closed. Also, the blood return valve 19, the blood merging valve 21 and the cleansing liquid discharge valve 22 have been closed at the previous step S2. Starting from this condition, the slow hemodiafiltration apparatus is allowed to stand for a period of 20 seconds. If after the lapse of this period of 20 seconds, the first to third pressure sensors 41, 44 and 47 detect that the respective pressures acting on the first to third membrane filters 40, 43 and 46 are, for example, -70 mmHg or lower, it is corroborated that ascertainment of circuit connection at step S2 is correct.

On the other hand, if the first to third pressure sensors 41, 44 and 47 detect within the period of 20 seconds that the respective pressures acting on the first to third membrane filters 40, 43 and 46 are of a value in excess of -70 mmHg, it is ascertained that the first blood processing unit 1 is not properly fluidly connected with any of the blood introducing passage 8, the blood return passage 9, the delivery passage 10 and the dialysis liquid introducing passage 11. Other than that, it can also be ascertained that the first air line 30 is properly fluidly connected with the first membrane filter 40 and the first pressure sensor 41, the second air line 31 is properly fluidly connected with the second membrane filter 43 and the second pressure sensor 44 and the third air line 32 is properly fluidly connected with the third membrane filter 46 and the third pressure sensor 47. In such case, after the attendant worker has connected respective passages 8, 9 and 10 and the first to third air lines 30, 31 and 32 with the first blood processing unit 1, the sequence of steps S1 to S3 are executed again.

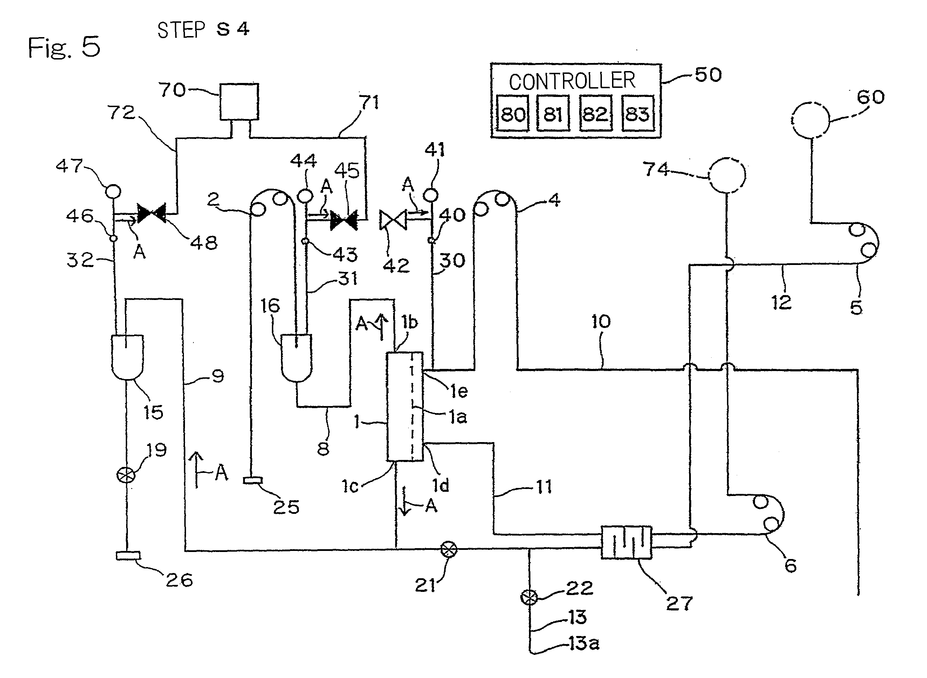

Thereafter, at step S4 shown in Fig. 5, the first air valve 42 is set in an opened condition for a period of 10 seconds. By so doing, air A flows in the fluid circuit as shown by the arrow headed lines and the pressure inside the fluid circuit changes from the negative pressure to the atmospheric pressure. Thus, in the event that subsequent to change of the opened or closed condition the first to third pressure sensors 41, 44 and 47 detect that the respective pressures acting on the first to third membrane filters 40, 43 and 46 attain a value higher than, for example, -50 mmHg, it is ascertained even at step S4 that the fluid circuit is properly connected. On the other hand, in the event that it is detected that the pressure acting on one of the first to third membrane filters 40, 43 and 46, for example, the second membrane filter 43 is of a value lower than -50 mmHg, it is suspected that no air A flow in the second air line 31 because the second air line 31 or a portion of the blood introducing passage 8 downstream of the blood introducing pump 2 is blocked by, for example, clamp or forceps (pinch). In such case, the attendant worker has to perform the sequence of steps S 1 to S4 again after the blocked condition is released from the second air line 31.

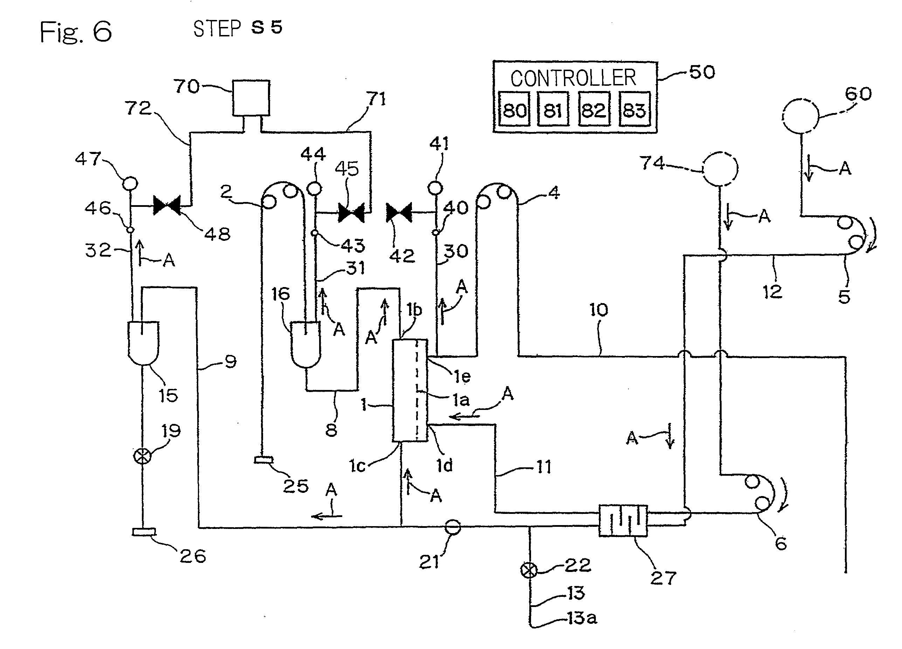

Then, at step S5 shown in Fig. 6, when the controller 50 causes the first to third air valves 42, 45 and 48 in the associated air lines 30, 31 and 32, the blood return valve 19 and the cleansing liquid discharge valve 22 to be closed, the blood merging valve 21 to be opened and the second pump 6 and the third pump 5 to be rotated in the normal direction, air A flows into a portion of the liquid replacement introducing passage 12 downstream of the third pump 5, a portion of the dialysis liquid introducing passage 11 downstream of the second pump 6, the first blood processing unit 1, a portion of the blood introducing passage 8 downstream of the blood introducing pump 2, the blood return passage 9, the first air line 30, the second air line 31 and the third air line 32 from the outside of the apparatus and, therefore, a positive pressure is developed.

At step S5, the second pump 6 and the third pump 5 are rotated in the normal direction at a flow rate of, for example, 40 ml/min. for a period of 30 seconds. If within this period of 30 seconds, the second pressure sensor 44 in the second air line 31 and the third pressure sensor 47 in the third air line 32 detect that respective pressures acting on the second membrane filter 43 and the third membrane filter 46 attains a value higher than, for example, +50 mmHg, the controller 50 causes the second pump 6 to halt. Also, if the first pressure sensor 41 in the first air line 30 detects that the pressure acting on the first membrane filter 40 attains a value higher than + 50 mmHg, the controller 50 causes the third pump 5 to halt.

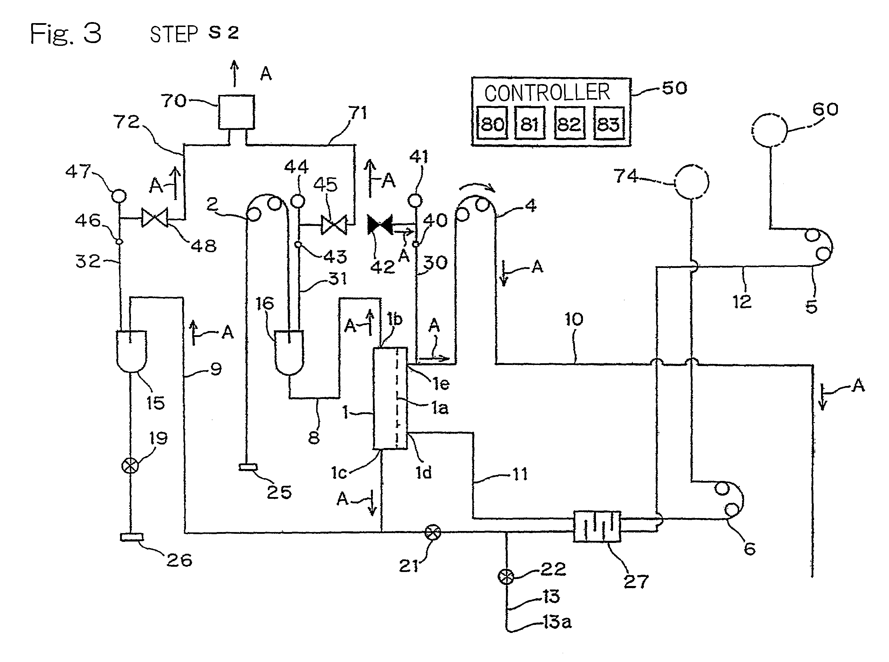

As hereinabove described, if the respective pressures (positive pressures) acting on the first to third membrane filters 40, 43 and 46 attain the second predetermined pressure value (for example, +50 mmHg or higher in the illustrated embodiment), it is ascertained that the first blood processing unit 1 is properly fluidly connected with the dialysis liquid introducing passage 11 and the liquid replacement introducing passage 12. At this step S5, whether or not particularly the dialysis liquid introducing passage 11 is properly fluidly connected with the first blood processing unit 1 is ascertained. In the event that at the time of installation of the first blood processing unit 1 in the slow hemodiafiltration apparatus, removal of a protective cap from the second connection port 1d of the first blood processing unit 1 is forgotten, since at step S2 shown in Fig. 3 for the purpose of ascertaining the fluid connection by the negative pressure, the negative pressure is developed in the first air line 30 even if connection between the dialysis liquid introducing passage 11 and the second connection port 1d is faulty, it has not been possible to ascertain whether or not the dialysis liquid introducing passage 11 and the second connection port 1d of the first blood processing unit 1 are properly fluidly connected with each other. At this step S5, however, since ascertainment of the connection by the positive pressure by the utilization of the air from the outside of the apparatus is carried out, that is, since a pressure can be applied by means of air from the outside of the apparatus from the dialysis liquid introducing passage 11 to the second connection port 1d of the first blood processing unit 1, it is possible to ascertain the fluid connection between the dialysis liquid introducing passage 11 and the second connection port 1d of the first blood processing unit 1.

On the other hand, if the first to third pressure sensors 41, 44 and 47 fail to detect the pressure exceeding the second predetermined pressure value (for example, +50 mmHg or higher in the illustrated embodiment), for example, if the pressure acting on the first membrane filter 40 does not attain a value exceeding +50 mmHg or higher, it is suspected that the fluid connection between the dialysis liquid introducing passage 11 and the first blood processing unit 1 is not properly done in the manner described hereinbefore. Also, if, for example, the pressure acting on the third membrane filter 46 does not attain a value exceeding +50 mmHg or higher, it is suspected that the liquid replacement introducing passage 12 is blocked by a clamp or the like. After such failure in circuit connection has been properly remedied, the attendant worker executes step S5 again.

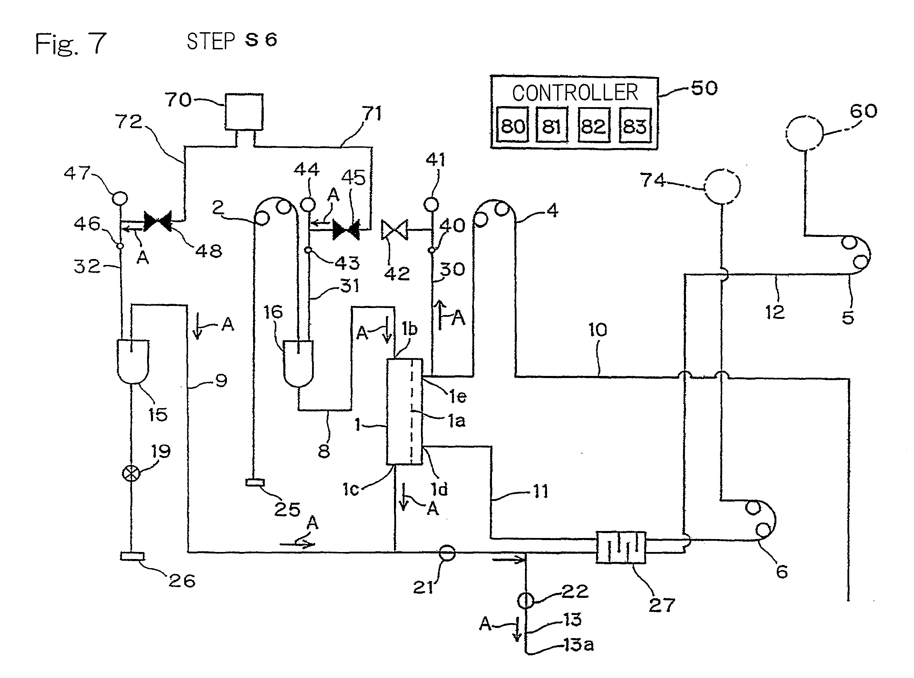

Finally, at step S6 shown in Fig. 7, when the controller 50 causes the blood merging valve 21 in the liquid replacement passage 12 and the cleansing liquid discharge valve 22 in the cleansing liquid discharge passage 13 to be opened for a period of 10 seconds and subsequently causes the blood merging valve 21 and the cleansing liquid discharge valve 22 to be closed and the first air valve 42 in the first air line 30 to be opened for a period of 10 seconds, the air A flows towards the liquid replacement passage 12 as shown by the arrow headed lines and is subsequently discharged to the outside of the apparatus through the cleansing liquid discharge port 13a after having flowed through the blood merging valve 21 and the cleansing liquid discharge valve 22, and, at the same time, the air A is discharged to the outside of the apparatus through the first air valve 42 by way of the first air line 30. Accordingly, the pressure inside the fluid circuit changes from the positive pressure (step S5) down to the atmospheric pressure, for example, +20 mmHg or lower. In the event that although the blood merging valve 21, the cleansing liquid discharge valve 22 and the first air valve 42 are opened for a period of 10 seconds, the respective pressures acting on the first to third membrane filters 40, 43 and 46 are not reduced down to +20 mmHg or lower, it is suspected, for example, that the liquid replacement passage 12 or the cleansing liquid discharge passage 13 may be blocked by a clamp or the like. After the attendant worker has remedied such failure in circuit connection, the connection ascertaining procedure is again initiated from step S5 shown in Fig. 6. At this time, if the first blood processing unit 1 is of a wet type, in which filling liquid has been filled inside the first blood processing unit beforehand, the filling liquid urges the air inside the separating membrane 1a to flow from upper side downwardly through the first blood processing unit 1 mounted vertically, until such air is discharged from the blood outlet 1c and, accordingly, it can be avoided that air may be left remaining inside the separating membrane 1a. After step S6, the first to third air valves 42, 45 and 48 and all of the valves 19, 21 and 22 in the fluid circuit are closed. By so doing, the connection failure ascertaining procedure completes.

As hereinabove described, when by executing steps S1 to S6 shown respectively in Figs. 2 to 7, the negative pressure is first generated inside the fluid circuit (step S2), followed by generation of the positive pressure inside the fluid circuit (step S6), the status of fluid connection in the fluid circuit and the presence or absence of pumping equipments can be ascertained properly.

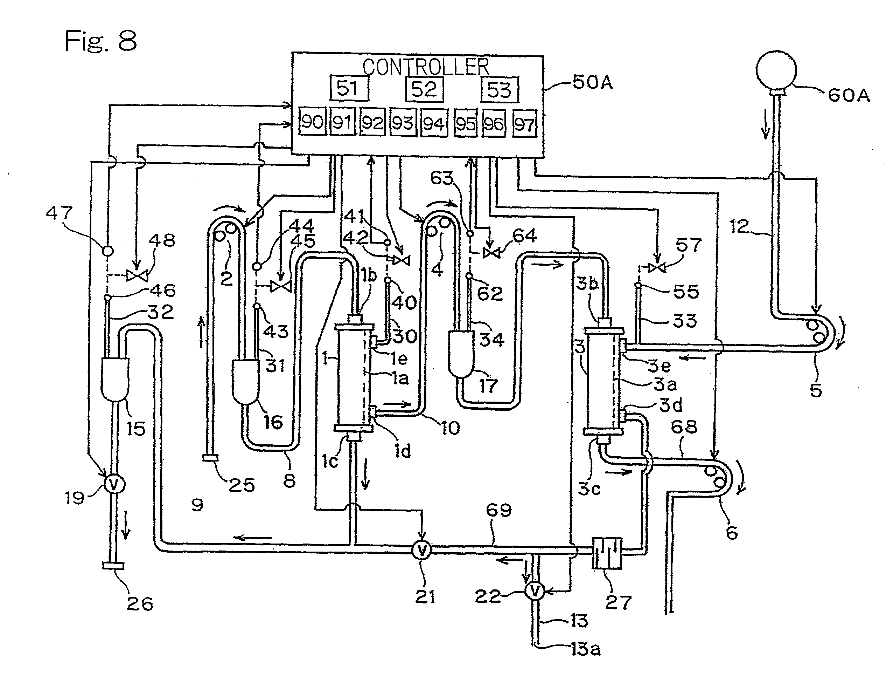

Hereinafter, the double filtration blood purification apparatus according to a second preferred embodiment of the present invention will be described in detail. Fig. 8 illustrates a schematic diagram showing a fluid circuit configuration, in which the slow hemodiafiltration apparatus shown in and described with particular reference to Fig. 1 is replaced with the double filtration blood purification apparatus. This double filtration blood purification apparatus is of a double filtration type and makes use of a first blood processing unit 1, which is a blood component separator for separating the blood into a blood cell component and a plasma component, and a second blood processing unit 3, which is a plasma component separator for separating the separated plasma component into a low molecular weight subcomponent and a high molecular weight subcomponent containing toxins.

Each of the first blood processing unit 1 and the second blood processing unit 3 is in the form of, for example, a cylindrical housing having a hollow fiber or flat plate, tubular separating membrane accommodated therein. The separating membrane 1a of the first blood processing unit 1 has a multiplicity of pores defined therein and having a pore size within the range of 0.1 to 0.5 µm, preferably about 0.2 µm and is employed in the form of a homogeneous microporous membrane, a microfiltration membrane or an asymmetric membrane made up of a porous support layer and a microporous structural layer. Although as a membrane 1 a for the first blood processing unit 1 various separating membranes have been well known, the use is preferred of the separating membrane having an excellent biocompatibility made of a copolymer of a polyvinyl alcohol (PVA) system, a copolymer of an ethylene vinyl alcohol (EVA) system, a cellulose derivative or polysulfone. On the other hand, the separating membrane 3a of the second blood processing unit 3 has a multiplicity of pores defined therein and having a pore size within the range of 0.01 to 0.04 micrometer, preferably about 0.02 µm and is employed in the form of, for example, a homogeneous microporous membrane, a microfiltration membrane or an asymmetric membrane made up of a porous support layer and a microporous structural layer. Although various separating membranes have been well known as a membrane 3a for the second blood processing unit 3, the use is preferred of the separating membrane having an excellent biocompatibility made of a copolymer of the EVA system or a cellulose derivative or polysulfone. In any event, in the illustrated embodiment, each of the first blood processing unit 1 and the second blood processing unit 3 is a wet type including a cylindrical housing having a multiplicity of tubular hollow fiber membranes accommodated therein and also having a filling liquid filled therein and disposed with its longitudinal axis oriented vertically. It is, however, to be noted that each of the first blood processing unit 1 and the second blood processing unit 3 may be of a dry type, in which no filling liquid is filled therein beforehand.

The first blood processing unit 1 is fluidly connected with a blood introducing passage 8 for introducing therethrough a blood, drawn from a blood introducing element 25 (an element that can be communicated with an ordinary blood collecting vessel such as, for example, a shunt and a syringe needle, or a blood reservoir), into the first blood processing unit 1, and the blood introducing passage 8 is provided with, from an upstream side thereof, a blood introducing pump 2 and then with a blood drip chamber 16. Subsequently the blood is, after the pressure thereof has been increased by the blood introducing pump 2, supplied dropwise into the blood drip chamber 16. Then, the blood so supplied dropwise from the blood drip chamber 16 is introduced into the first blood processing unit 1 by way of a blood inlet 1b, defined in an upper (upstream) end portion thereof, so that the blood can be separated by the separating membrane 1a into a blood cell component and a plasma component.

The first blood processing unit 1 is also fluidly connected with a blood return passage 9 through which the blood cell component separated from the blood in the manner described above is returned to a patient's body, and this blood return passage 9 is fluidly connected with a return blood drip chamber 15 and then with a blood return valve 19, both disposed in an upstream portion of the blood return passage 9 with respect to the direction of flow of the separated blood cell component towards the patient's body. The separated blood cell component emerges outwardly from the first blood processing unit 1 by way of a blood outlet 1c defined in a lower (downstream) end portion of such first blood processing unit 1 and is then supplied into the return blood drip chamber 15. Thereafter, the blood cell component supplied dropwise from the return blood drip chamber 15 is, after having been introduced to a blood delivery element 26 (an element that can be communicated with a shunt or an intravenous drip kit) by way of a blood return valve 19 then opened, returned to the patient's body.

The second blood processing unit 3 is fluidly connected with the first blood processing unit 1 through the delivery passage 10. The delivery passage 10 extends from a second connection port 1d, which is defined in a portion of the side surface of the first blood processing unit 1 proximate to the blood outlet 1c and forms a plasma outlet, to a plasma inlet 3b defined in an upper (upstream) end portion of the second blood processing unit 3. The delivery passage 10 is provided with a first pump 4 for the plasma delivery and a plasma drip chamber 17 in this order from an upstream side. The plasma component emerging outwardly from the second connection port 1d of the first blood processing unit 1 is, after the pressure thereof has been increased by the first pump 4, introduced into the plasma drip chamber 17. Subsequently, the plasma component supplied dropwise from the plasma drip chamber 17 is introduced into the second blood processing unit 3 from above by way of a plasma inlet 3b so that the plasma component can be separated into a low molecular weight subcomponent and a high molecular weight subcomponent.

The second blood processing unit 3 has a lower (downstream) end portion formed with a first plasma component outlet 3c for delivering the separated high molecular weight subcomponent and also has a portion of the side surface thereof proximate to the first plasma component outlet 3c formed with a second plasma component outlet 3d for delivering the separated low molecular weight subcomponent. The first plasma component outlet 3c of the second blood processing unit 3 is fluidly connected with a plasma discharge passage 68 for discharging the separated plasma high molecular weight component to the outside of the apparatus, and this plasma discharge passage 68 is provided with a second pump 6 for the plasma delivery. The plasma high molecular weight component emerging from the first plasma component outlet 3c is, after the pressure thereof has been increased by the second pump 6, discharged to the outside of the apparatus through the plasma discharge passage 68.

On the other hand, a plasma return passage 69 for returning the separated low molecular weight subcomponent back to the patient's body extends from the second plasma component outlet 3d of the second blood processing unit 3 and is merged with the blood return passage 9 at a location between the blood outlet 1c of the first blood processing unit 1 and the return blood drip chamber 15. This plasma return passage 69 is provided with a heater 27 for heating the plasma low molecular weight component to be returned to the patient's body and a blood merging valve 21 for selectively establishing or interrupting communication. When the blood merging valve 21 is closed, the plasma low molecular weight component emerging from the second plasma component outlet 3d is returned to the patient's body through the plasma return passage 69 and the blood return passage 9. Also, a portion of the plasma return passage 69 between the second plasma component outlet 3d of the second blood processing unit 3 and the blood merging valve 21 is fluidly connected with a cleansing liquid discharge passage 13. This cleansing liquid discharge passage 13 is provided with a cleansing liquid discharge valve 22 for selectively establishing or interrupting the communication with the outside of the apparatus, and, when during the priming procedure taking place, this cleansing liquid discharge valve 22 is opened, the cleansing liquid is discharged to the outside of the apparatus through a cleansing liquid discharge port 13a.

A portion of a side surface of the second blood processing unit proximate to the liquid replacement inlet 3e is fluidly connected with a liquid replacement introducing passage 12 for introducing a liquid replacement from a liquid replacement supply source 60A for the liquid replacement into the second blood processing unit 3. This liquid replacement introducing passage 12 is provided with a third pump 5 for introducing the liquid replacement. The liquid replacement emerging the liquid replacement supply source 60A is, after the pressure thereof has been increased by the third pump 5, introduced into the second blood processing unit 3 by way of the liquid replacement inlet 3e.

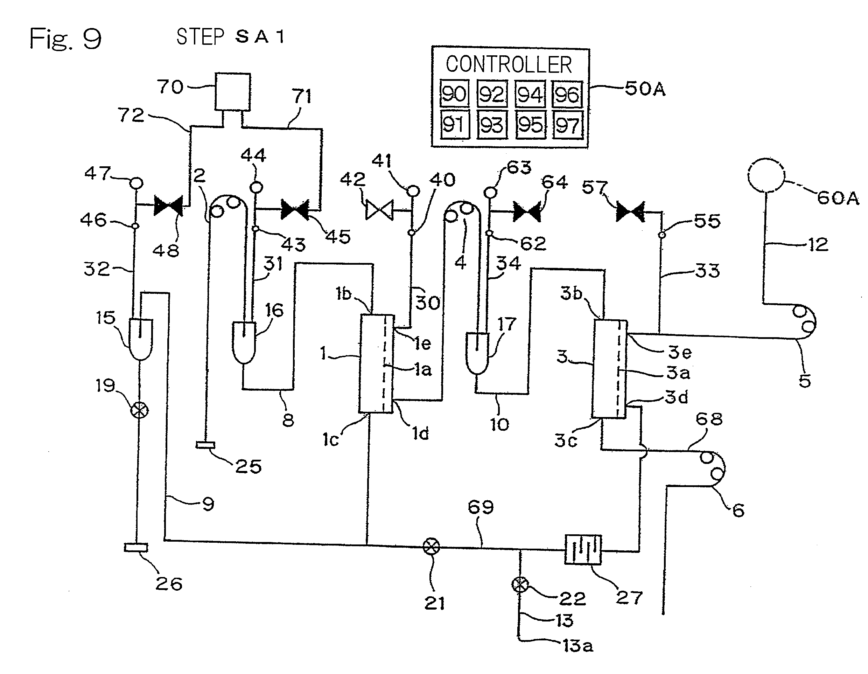

A portion of the first blood processing unit 1 proximate to the blood inlet 1b is provided with a first connection port 1e, to which a first air line 30 is fluid connected. This first air line 30 is provided with a first membrane filter 40, a first pressure sensor 41 for detecting a membrane pressure of the separating membrane 1a of the first blood processing unit 1, and a first air valve 42 for selectively establishing or interrupting communication with the outside of the apparatus. By causing the air valve 42 to be opened, air can be introduced or discharged between the first blood processing unit 1 and the outside of the apparatus. Also, as is the case with the slow hemodiafiltration apparatus shown in and described with particular reference to Fig. 1, even in this double filtration blood purification apparatus, the blood drip chamber 16 is fluidly connected with a second air line 31, a second membrane filter 43, a second pressure sensor 44 and a second air valve 45, and the return blood drip chamber 15 is fluidly connected with a third air line 32, a third membrane filter 46, a third pressure sensor 47 and a third air valve 48. Also, the second air valve 45 and the third air valve 48 are fluidly connected with an air pump 70 (Fig. 9) through respective air tubes 71 and 72.

A portion of the liquid replacement introducing passage 12 between the third pump 5 and the second blood processing unit 3 is fluidly connected with a fourth air line 33. This fourth air line 33 is provided with a fourth membrane filter 55 and a fourth air valve 57 for selectively establishing or interrupting communication with the outside of the apparatus. Also, the plasma drip chamber 17 is fluidly connected with a fifth air line 34, which is provided with a fifth membrane filter 62, a fifth pressure sensor 63 for detecting the pressure inside the plasma drip chamber 17 and a fifth air valve 64 for selectively establishing or interrupting communication with the outside of the apparatus.

The double filtration blood purification apparatus of the construction described hereinabove is provided with a controller 50A. This controller 50A has a pump drive unit 51 for driving the blood introducing pump 2, the first pump 4, the second pump 6, the third pump 5 and the air pump 70 (Fig. 9), a circuit valve drive unit 52 for selectively opening or closing the blood return valve 19, the blood merging valve 21 and the cleansing liquid discharge valve 22, and an air valve drive unit 53 for selectively opening or closing the first to fifth air valves 42, 45, 48, 57 and 64, all built therein. This controller 50A controls the pump drive unit 51 and the valve drive unit 52, based on respective detected pressure signals fed from the first, second, third and fifth pressure sensors 41, 44, 47 and 63.

The controller 50A also has a blood side connection ascertaining mode setting section 90, a blood side connection ascertaining section 91, a plasma side connection ascertaining mode setting section 92, a plasma side connection ascertaining section 93, a liquid replacement side connection ascertaining mode setting section 94, a liquid replacement side connection ascertaining section 95, a discharge side connection ascertaining mode setting section 96 and a discharge side connection ascertaining section 97. In this double filtration blood purification apparatus, when a failure in circuit connection is to be ascertained, the blood side connection ascertaining mode setting section 90 is operable to control not only selective opening or closure of each of the blood return valve 19, the blood merging valve 21, the first air valve 42, the second air valve 45, the third air valve 48 and the fifth air valve 64, but also rotation of each of the first pump 4 and the air pump 70, to thereby generate a negative pressure inside a blood circuit, including the blood introducing passage 8, the blood return passage 9 and the air line, and the first blood processing unit 1. On the other hand, the blood side connection ascertaining section 91 is operable to ascertain that the blood passages 8 and 9 are connected with the first blood processing unit 1, when the negative pressure referred to above attains a first predetermined pressure value within a predetermined time and is constituted by, for example, an alarm and/or an indicator lamp. At steps SA1 to SA4 shown respectively in Figs. 9 to 12 and as will be described in detail later, the attendant worker can be informed of the failure, occurring in circuit connection, by means of sounds generated by the alarm and/or indicator lamp caused by the display lamp in the event of the failure being found present in circuit connection.

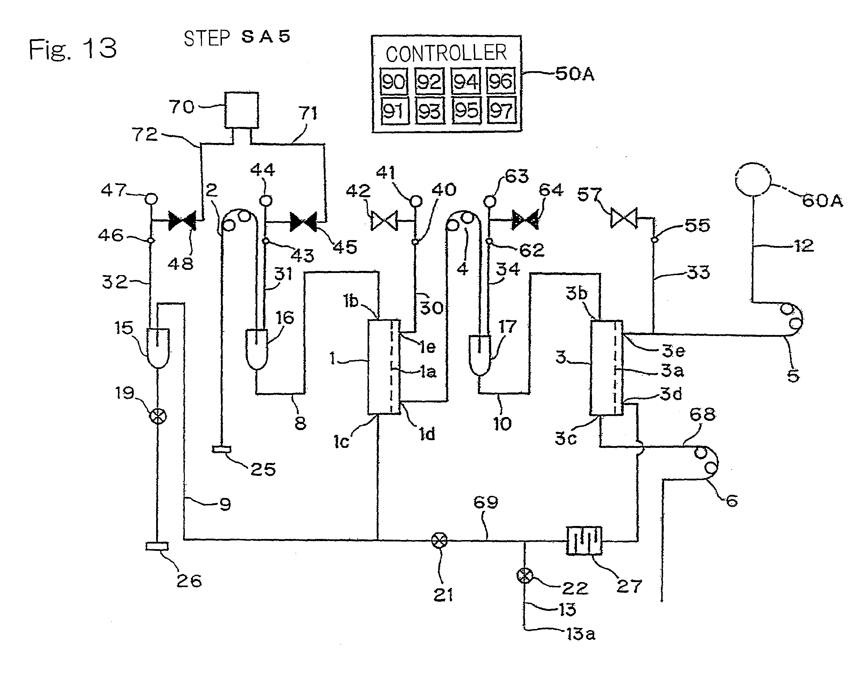

Also, the plasma side connection ascertaining mode setting section 92 is operable to control the selective opening or closure of the first air valve 42 and the fourth air valve 57 and to control the rotation of the first pump 4 to thereby generate a negative pressure inside the delivery passage 10, the liquid replacement introducing passage 12 and the second blood processing unit 3 and, also, to generate a positive pressure inside the circuit including the blood introducing passage 8, the blood return passage 9 and the air line, and inside the first blood processing unit 1. On the other hand, the plasma side connection ascertaining section 93 is operable to ascertain that the delivery passage 10 is connected with the first blood processing unit 1, when the negative referred to above attains a second predetermined value within a predetermined time, and is constituted by, for example, an alarm and/or indicator lamp. At steps SA5 to SA7 shown respectively in Figs. 13 to 15 and as will be described in detail later, the attendant worker can be informed of the failure, occurring in circuit connection, by means of sounds generated by the alarm and/or indicator lamp caused by the display lamp in the event of the failure being found present in circuit connection.

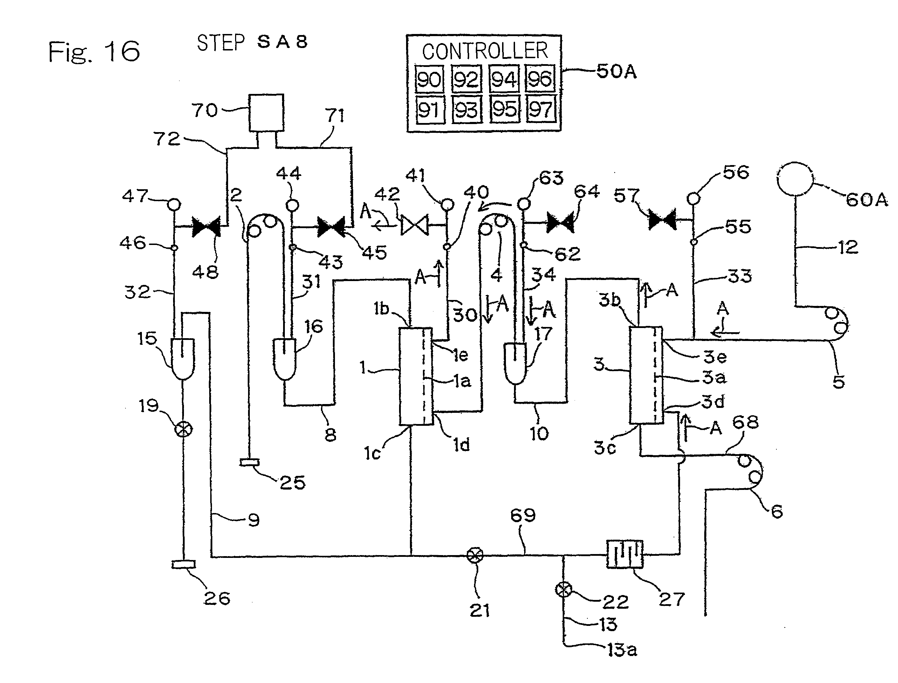

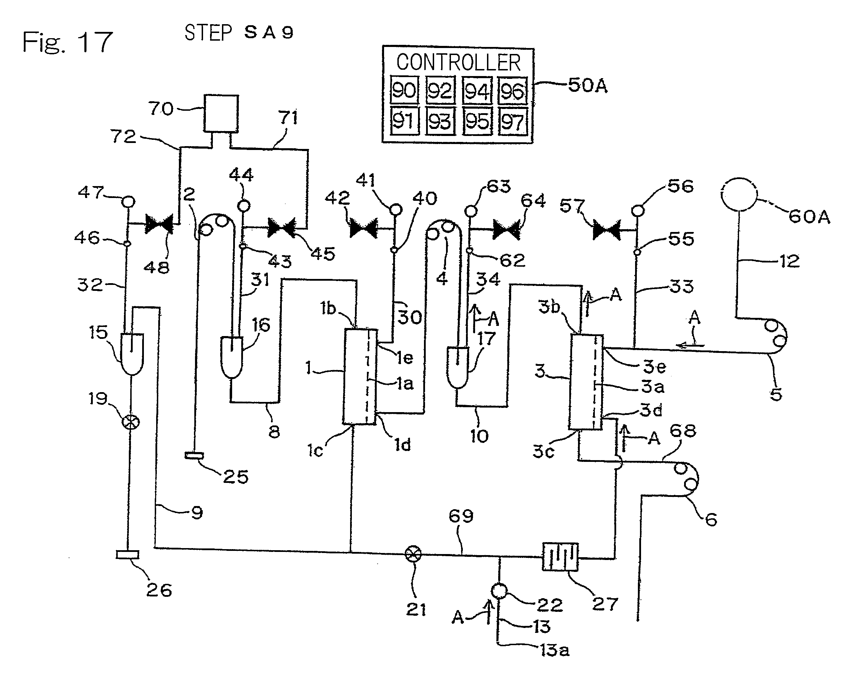

The liquid replacement side connection ascertaining mode setting section 94 is operable to control the selective opening or closure of the first air valve 42 and the cleansing liquid discharge valve 22 and to control the rotation of the first pump 4, to thereby generate a negative pressure inside the circuit including the delivery passage 10, the liquid replacement introducing passage 12 and the plasma return passage 69, and the second blood processing unit 3. On the other hand, the liquid replacement connection ascertaining section 95 is operable to ascertain that the blood return passage 69 and the second blood processing unit 3 are fluidly connected with each other, when the negative pressure referred to above attains a third predetermined pressure value within a predetermined time, and is constituted by, for example, an alarm and/or indicator lamp. At steps SA8 and SA9 shown respectively in Figs. 16 to 17 and as will be described in detail later, the attendant worker can be informed of the failure, occurring in circuit connection, by means of sounds generated by the alarm and/or indicator lamp caused by the display lamp in the event of the failure being found present in circuit connection.

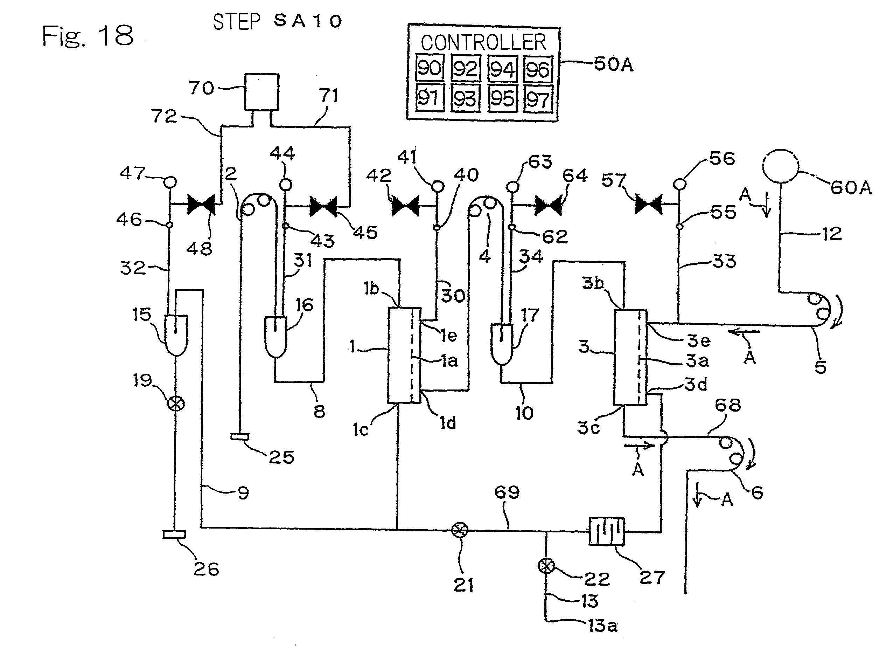

Furthermore, the discharge side connection ascertaining mode setting section 96 is operable to control the rotation of the second pump 6 and the third pump 5 to generate a predetermined pressure inside the circuit including the liquid replacement introducing passage 12 and the plasma discharge passage 68, and the second blood processing unit 3. On the other hand, the discharge side connection ascertaining section 97 is operable to ascertain that the plasma discharge passage 68 and the second blood processing unit 3 are fluidly connected with each other due to the above mentioned pressure retained at a constant value for a predetermined time, and is constituted by, for example, an alarm and/or indicator lamp. At step SA10 shown in Fig. 18 and as will be described in detail later, the attendant worker can be informed of the failure, occurring in circuit connection, by means of sounds generated by the alarm and/or indicator lamp caused by the display lamp in the event of the failure being found present in circuit connection.

Hereinafter, the procedure to ascertain the presence of a failure in circuit connection in the double filtration blood purification apparatus of the construction shown in and described with particular reference to Fig. 8 will now be described with particular reference to steps SA1 to SA10 shown respectively in Figs. 9 to 18. It is to be noted in steps SA1 to SA10 shown respectively in Figs. 9 to 18, the arrow headed lines indicate the directions of flow of air A.

At the outset, at step SA1 shown in Fig. 9, the controller 50A is activated in response to a start signal fed from the outside. By this controller 50A, the first air valve 42 is opened, the second to fifth air valves 45, 48, 57 and 64, the blood return valve 19, the blood merging valve 21 and the cleansing liquid discharge valve 22 are closed.

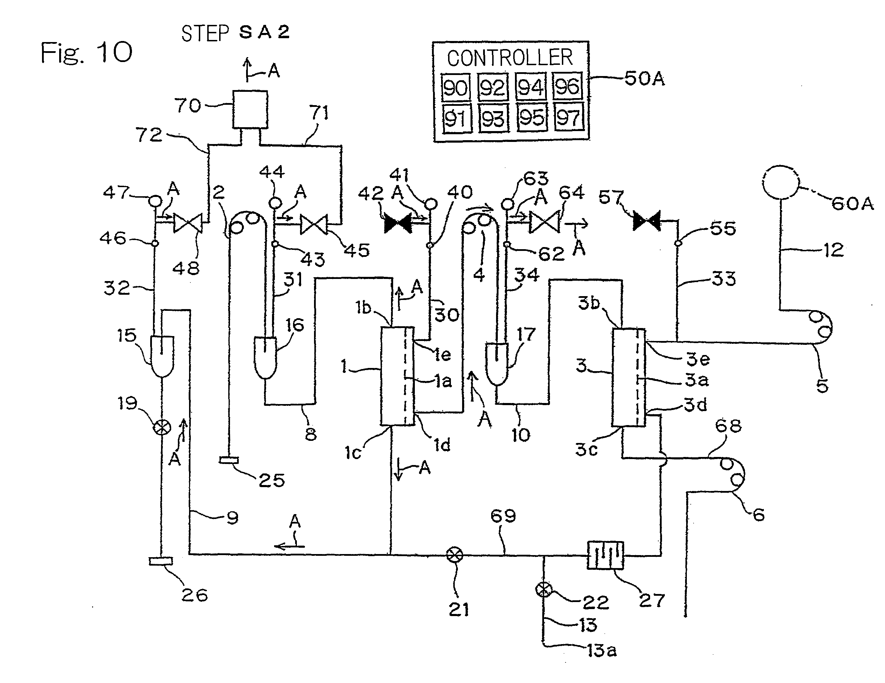

Subsequently, at step SA2 shown in Fig. 10, when by the controller 50A, the second air vale 45, the third air valve 48 and the fifth air valve 64 are opened, the first air valve 42 is closed, the air pump 70 is rotated in a reverse direction and the first pump 4 is rotated in a normal direction, air A flows in directions as indicated by the arrow headed lines and, therefore, a negative pressure is developed inside a portion of the blood introducing passage 8 downstream of the blood introducing pump 2, a portion of the blood return passage 9 upstream of the blood return valve 19, a portion of the delivery passage 10 upstream of the first pump 4 and inside the first blood processing unit 1. At this step SA2, the air A is finally discharged to the outside of the apparatus through an air discharge port (not shown) provided in the air pump 70, and the fifth air valve 64.

At this step SA2, the air pump 70 and the first pump 4 are rotated for a period of, for example, 30 seconds. If within this period of 30 seconds, the second pressure sensor 44 detects that the pressure acting on the second membrane filter 43 attains a first predetermined pressure value, for example, -80 mmHg or lower, and the third pressure sensor 47 detects that the pressure acting on the third membrane filter 46 attains a value of -80 mmHg, the controller 50A causes the air pump 70 to halt. On the other hand, if within the period of 30 seconds, the first pressure sensor 41 detects that the pressure (negative pressure) acting on the first membrane filter 40 attains a value equal to or lower than -80 mmHg, the controller 50 causes the first pump 4 to halt. In this way, in the event that the first to third pressure sensors 41, 44 and 47 detect the first predetermined pressure value within the period of 30 seconds, it can be ascertained that the first blood processing unit 1 is properly fluidly connected with the blood introducing passage 8, the blood return passage 9, the delivery passage 10 and the first air line 30 with no air leakage occurring therein. Also, other than that, it can also be ascertained that the first air line 30 is properly fluidly connected with the first membrane filter 40 and the first pressure sensor 41, the second air line 31 is properly fluidly connected with the second membrane filter 43 and the second pressure sensor 44 and the third air line 32 is properly fluidly connected with the third membrane filter 46 and the third pressure sensor 47.

On the other hand, in the event that any one of the first to third pressure sensors 41, 44 and 47 fails to detect the first predetermined pressure value (for example, -80 mmHg in the illustrated embodiment) within the period of 30 seconds, it is ascertained that the first blood processing unit 1 is not properly fluidly connected with the blood introducing passage 8, the blood return passage 9 and the delivery passage 10. Other than that, it can be also ascertained that the first air line 30 is not properly fluidly connected with the first membrane filter 40 and the first pressure sensor 41, the second air line 31 is not properly fluidly connected with the second membrane filter 43 and the second pressure sensor 44, and the third air line 32 is not properly fluidly connected with the third membrane filter 46 and the third pressure sensor 47. Once the failure in circuit connection is so ascertained, after the attendant worker has removed such failure in circuit connection, steps SA1 to SA2 are sequentially carried out again, followed by reconfirmation of whether the fluid circuit is properly fluid connected.

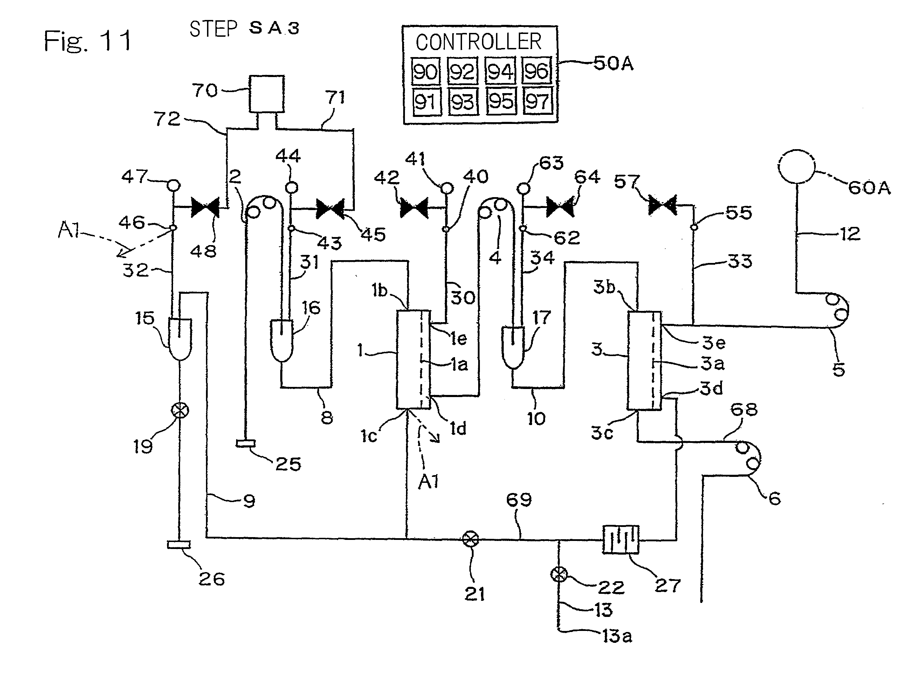

Thereafter, at step SA3 shown in Fig. 11, by the controller 50A, the second air valve 45, the third air valve 48 and the fifth air valve 64 are closed so that all of the air valves are also closed. On the other hand, the blood return valve 19, the blood merging valve 21 and the cleansing liquid discharge valve 22 have been closed during the previous step SA2. Starting this condition, the double filtration blood purification apparatus is allowed to stand for a period of 20 seconds. If after the lapse of this period of 20 seconds the first to third pressure sensors 41, 44 and 47 detect that the respective pressures acting on the first to third membrane filters 40, 43 and 46 are -70 mmHg or lower, it is corroborated that ascertainment of circuit connection at step S2 is correct.

On the other hand, if within this period of 20 seconds the third pressure sensor 47, for example, detects that the pressure acting on the third membrane filter 46 is higher than -70 mmHg, it may be suspected that since the air A1 leaks from a connection between the third air line 32 and the third membrane filter 46, the third membrane filter 46 is not properly mounted on the third air line 32, and that since the air A1 leaks from a connection between the first blood processing unit 1 and the blood return passage 9 the blood return passage 9 is not properly fluidly connected with the first blood processing unit 1. After the attendant worker has removed such failure in circuit connection, the procedure starting from SA1 shown in Fig. 9 has to be again performed to ascertain the presence or absence of the failure in circuit connection. Thus, by conducting this step SA3, the ascertainment of the presence or absence of the failure in circuit connection performed at step SA2 can be corroborated.

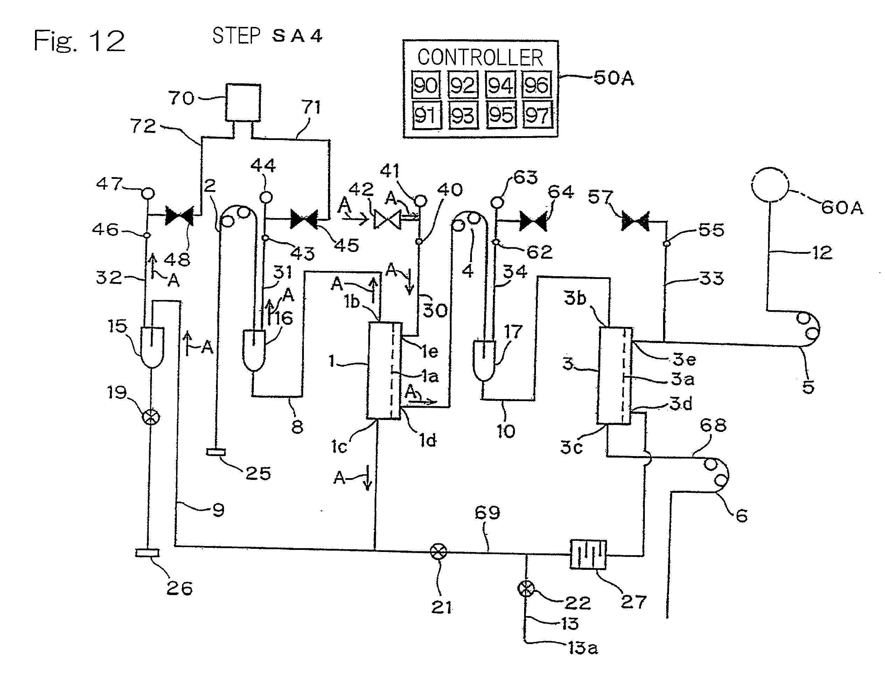

At step SA4 shown in Fig. 12, when by the controller 50A only the first air valve 42 is opened from the condition assumed during step SA3, air from the outside of the apparatus flows in the first air line 40 as shown by the arrow headed lines and, as a result, the pressure inside the first blood processing unit 1, the blood introducing passage 8 and the blood return passage 9 changes from the negative pressure to the atmospheric pressure. If within this period of 20 seconds the first to third pressure sensors 41, 44 and 47 detect the respective pressures acting on the first to third membrane filters 40, 43 and 46 attains a value in excess of -50 mmHg, the circuit is ascertained as properly connected and the ascertainment of the presence or absence of the failure in circuit connection performed at step SA2 can be corroborated. On the other hand, if within this period of 20 seconds the respective pressures acting on the first to third membrane filters 40, 43 and 46, for example, the second membrane filter 43, are detected to be lower than -50 mmHg, it is suspected that air fail to flow into the second air line 31 since the second air line 31 or the blood introducing passage 8 is blocked by a clamp or the like. After the attendant worker has remedied this blockage, the presence of a failure in circuit connection has to be again carried out, starting from step SA1 shown in Fig. 9.

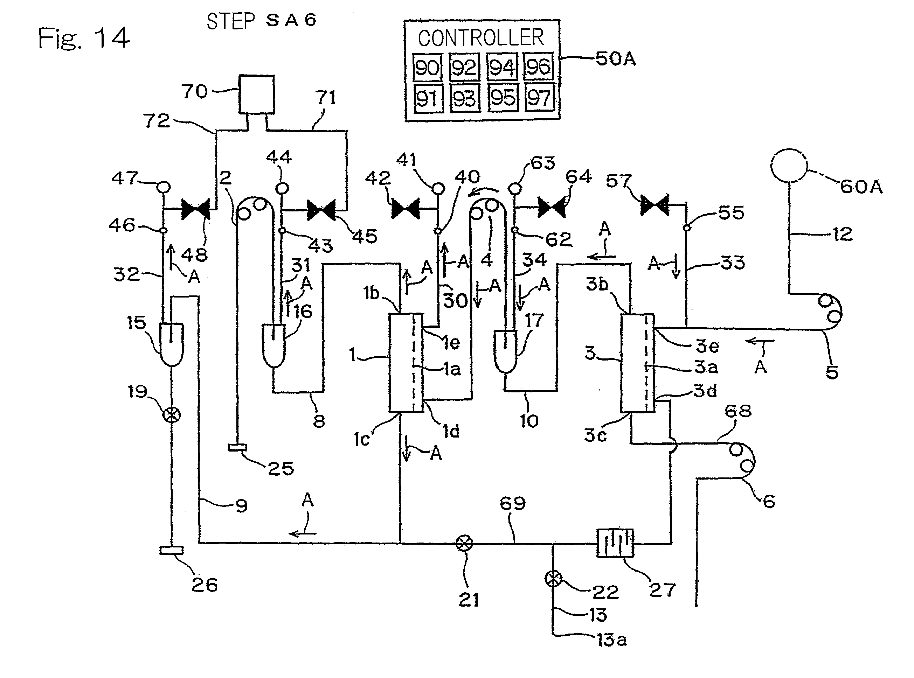

Then at step SA5 shown in Fig. 13, by the controller 50A, the first air valve 42 and the fourth air valve 57 are opened. After the first air valve 42 and the fourth air valve 57 are kept opened for a period of, for example, 5 seconds, step SA6 shown in Fig. 14 is carried out. At step SA6, when by the controller 50A, all of the air valve, including the first to fifth air valves 42, 45, 48, 57 and 64, the blood return valve 19, the blood merging valve 21 and the cleansing liquid discharge valve 22 are closed and the first pump 4 is rotated in the reverse direction, a negative pressure is developed inside a portion of the delivery passage downstream of the first pump 4, the second blood processing unit 3, a portion of the liquid replacement introducing passage 12 downstream of the third pump 5, the fourth air line 33 and the fifth air line 34 and, on the other hand, a positive pressure is developed inside a portion of the delivery passage 10 upstream of the first pump 4, the first blood processing unit 1, a portion of the blood introducing passage 8 downstream of the blood introducing pump 2, a portion of the blood return passage 9 upstream of the blood return valve 19 and the first to third air lines 30, 31 and 32.

At this step SA6, the plasma introducing pump 4 is rotated in the reverse direction for a period of 30 seconds at a flow rate of, for example, 30 ml/min. If within this period of 30 seconds, the fifth pressure sensor 63 detects that the pressure acting on the fifth membrane filter 62 attains the second predetermined pressure value (for example, -50 mmHg), the first pump 4 is halted by the controller 50A. At this time, the first pressure sensor 41 is relied on to ascertain whether or not the pressure acting on the first membrane filter 40 has attained +20 mmHg. If so ascertained, it can be ascertained that the delivery passage 10 is properly fluidly connected with the first blood processing unit 1. On the other hand, if not so ascertained, it can be ascertained that the delivery passage 10 is not properly fluidly connected with the first blood processing unit 1 or that portion of the delivery passage 10 upstream of the first pump 4 is blocked by a clamp or the like. In such case, after the attendant worker has remedies this failure, the ascertainment of the presence or absence of the failure in circuit connection is again performed, starting from SA5.

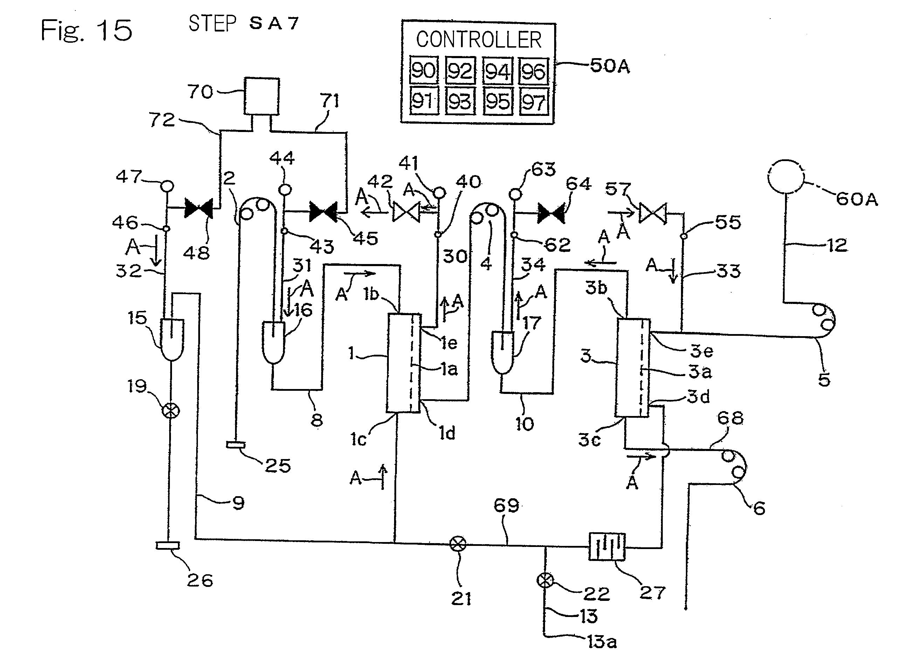

Thereafter, at step SA7 shown in Fig. 15, when by the controller 50A the first air valve 42 and the fourth air valve 57 are opened, the air A inside the blood introducing passage 8, the blood return passage 9 and the first blood processing unit 1, all assuming the positive pressure then, change from the positive pressure to the atmospheric pressure as a result of the air A flowing into the first air line 30. Also, as the air A flows in the fourth air line 33 then assuming the negative pressure, the air A also flows into the fifth air line 34 through the second blood processing unit 3 and then through the plasma drip chamber 17, changing the negative pressure to the atmosphere. The first air valve 42 and the fourth air valve 57 are opened for a period of, for example, 15 seconds. If within this period of 15 seconds, the fifth pressure sensor 63 detects that the pressure acting on the fifth membrane filter 62 attains a value in excess of, for example, -20 mmHg or higher, it can be ascertained that the fourth air line 33, the liquid replacement introducing passage 12 and the delivery passage 10 are properly fluidly connected with the second blood processing unit 3. On the other hand, if the pressure acting on the fifth membrane filter 62 did not attain a value in excess of -20 mmHg or higher, the ascertainment of the presence of a failure in circuit connection has to be performed again, starting from step SA5 shown in Fig. 13 after the attendant worker has connected properly the fourth air line 33 with the fourth membrane filter 55 and the fourth air valve 57 and has also connected properly the liquid replacement introducing passage 12 and the delivery passage 10 with the second blood processing unit 3.

Following step SA7 and at step SA8 shown in Fig. 16, when by the controller 50A, the fourth air valve is closed and the plasma introducing pump 4 is rotated in the reverse direction, the air A flows into a portion of the liquid replacement introducing passage 12 downstream of the third pump 5, a portion of the plasma return passage 69 upstream of the blood merging valve 21 and a portion between the second blood processing unit 3 and a downstream portion of the first pump 4 in the delivery passage 10 to cause a negative pressure developed therein. The first pump 4 is, by the controller 50A, rotated for a period of 20 seconds at a flow rate of, for example, 60 ml/min. At the time the fifth pressure sensor 63 detects that the pressure (negative pressure) acting on the fifth membrane filter 62 attains a value of, for example, -50 mmHg or lower this period of 20 second, the first pump 4 is halted by the controller 50A.

After during the previous step SA8 the fifth pressure sensor 63 has detected the negative pressure of -50 mmHg or lower and the first pump 4 has therefore been halted, step SA9 shown in Fig. 7 is executed. At step SA9, when by the controller 50A the first air valve 42 is closed to close all of the air valves 42, 45, 48, 57 and 64 and the cleansing liquid discharge valve 22 is opened, the air from the outside of the apparatus flows into the cleansing liquid discharge passage 13 and, as a result, the respective pressures inside the plasma return passage 69, the second blood processing unit 3, the liquid replacement introducing passage 112 and the delivery passage 10 changes from the negative pressure to the atmospheric pressure.

If after the valve opening and closure have been set in the manner as hereinabove described, for example, after a period of 10 second, the fifth pressure sensor 63 fails to detect that the pressure acting on the fifth membrane filter 62 attain a value higher than, for example, -20 mmHg or higher, it may be suspected that a trouble has occurred in the cleansing liquid discharge valve 22, the cleansing liquid discharge passage 13 is blocked by a clamp or the like, the plasma return passage 69 has not been properly fluidly connected with the second blood processing unit 3, and/or the air A has been unable to flow. Accordingly, after the attendant work has remedied the failure, the ascertainment of the presence of a failure in circuit connection is again executed, starting from step SA5 shown in Fig. 13.

Finally, at step SA10 shown in Fig. 18, when the controller 50A causes the cleansing liquid discharge valve 22 to be closed and the second pump 6 and the third pump 5 to be simultaneously rotated in the normal direction, the air A after having flowed from the outside of the apparatus into the liquid replacement introducing passage 12 as shown by the arrow headed lines and then flowed into the second blood processing unit 3 with the pressure thereof having increased by the third pump 5, and then into the plasma discharge passage 68 by way of the first plasma component outlet 3c, finally being discharged to the outside of the apparatus through the second pump 6. The second pump 6 and the third pump 5 are halted by the controller 50A after having been rotated in the normal direction for a period of 20 seconds at a flow rate of, for example, 40 ml/min. When after the second pump 6 and the third pump 5 having been halted, the pressure acting on the fifth membrane filter 62 is detected to be a value higher than, for example, 50 mmHg, it may be suspected that the plasma discharge passage 68 is not properly fluidly connected with the second blood processing unit 3 or the plasma discharge passage 68 is blocked by a clamp or the like. In such case, the attendant worker should take a proper remedy and, thereafter, the ascertainment of the presence of a failure in circuit connection is carried out again, starting from step SA5 shown in Fig. 13. After this step SA10 has been executed, the fourth air valve 57 and the cleansing liquid discharge valve 22 are temporarily opened, followed by closure of all of the first to fifth air valves 42, 45, 48, 57 and 64 and all of the circuit valves 19, 21 and 22. In this way, the connection failure ascertaining procedure completes.

When as hereinabove described the sequence of steps SA1 to SA10, shown respectively, in Figs. 9 to 18, are executed to generate the negative pressure inside the fluid circuit fluidly connected with the first blood processing unit 1 (step SA2), then generate the positive pressure inside the fluid circuit connected with the first blood processing unit 1 and, on the other hand, the negative pressure inside the fluid circuit connected with the second blood processing unit 3 (step SA6), subsequently generate the negative pressure inside the fluid circuit fluidly connected with the second blood processing unit 3 (step SA8), and finally maintaining the internal pressure of the liquid replacement introducing passage 12 and the plasma discharge passage 69 at a constant value, the presence of a failure in circuit connection and, also, whether or not the various pumps are mounted in the fluid circuit can be assuredly ascertained.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention.

By way of example, although the connection failure ascertaining method in the fluid circuit according to the present invention has been shown and described in connection with the preferred embodiments of the slow hemodiafiltration apparatus and the double filtration blood purification apparatus, respectively, the connection failure ascertaining method of the present invention can be applied not only to a dialyzing apparatus such as, for example, any one of the slow hemodiafiltration apparatus and the double filtration blood purification apparatus, but also to any other blood processing apparatus such as, for example, a plasma exchange apparatus and a plasma component adsorbing apparatus.

Accordingly, such changes and modifications are, unless they depart from the scope of the present invention as delivered from the claims annexed hereto, to be construed as included therein.

## Claims

1. A blood purification apparatus which comprises:
a first blood processing unit 1 for separating a blood into a first blood component containing a useful subcomponent and a second blood component containing a harmful subcomponent;
a blood circuit including a blood introducing passage 8 for introducing therethrough the blood into the blood processing unit 1, a blood return passage 9 for returning the separated first blood component back to a patient's body, and a delivery passage 10 for delivering the separated second blood component from the first blood processing unit 1;
a first pump 4 disposed in the delivery passage 10 for delivering fluid;
a blood return valve 19 disposed in the blood return passage 9;
an air pump 70 fluidly connected with the blood introducing passage 8 and also with the blood return passage 9; and
a controller 50 for controlling the first pump 4, the air pump 70 and the blood return valve 19,
wherein the controller 50 includes:
a discharge side connection ascertaining mode setting section 80 for generating a negative pressure inside the blood circuit and also inside the first blood processing unit 1 by causing the blood return valve 19 to be closed and the blood circuit to be blocked and, also, causing the first pump 4 to be driven in a normal direction and the air pump 70 to be driven in a reverse direction, respectively, to discharge air from the blood circuit; and
a blood side connection ascertaining section 81 for ascertaining, when the negative pressure attains a first predetermined pressure value within a predetermined time, a fluid connection between the blood circuit and the first blood processing unit 1.

2. The blood purification apparatus as claimed in Claim 1, which is formed as a blood filtering and dialyzing apparatus further comprising a dialysis liquid introducing passage 11 for introducing a dialysis liquid into the first blood processing unit 1, and a second pump 6 disposed in the dialysis liquid introducing passage 11,
wherein the controller 50 comprises, in place of or in addition to the blood side connection ascertaining mode setting section 80 and the blood side ascertaining section 81:
an introduction side connection ascertaining mode setting section 82 for generating a positive pressure inside the first blood processing unit 1 by driving the second pump 6 in a normal direction to allow air to flow into the first blood processing unit 1; and
an introduction side connection ascertaining section 83 for ascertaining a fluid connection between the dialysis liquid introducing passage 11 and the first blood processing unit 1 when the positive pressure attains a second predetermined pressure value within a predetermined time.

3. The blood purification apparatus as claimed in Claim 1, which is formed as a double filtration blood purification apparatus further comprising a second blood processing unit 3, fluidly connected with the delivery passage 10, for separating the second blood component into a high molecular weight subcomponent and a low molecular weight subcomponent;
wherein the controller 50 comprises, in place of or in addition to the blood side connection ascertaining mode setting section 80 and the blood side connection ascertaining section 81:
an introduction side connection ascertaining mode setting section 82 for generating a positive pressure inside the first blood processing unit 1 by driving the first pump 4 in a reverse direction to allow air to flow into the first blood processing unit 1; and
an introduction side connection ascertaining section 83 for ascertaining a fluid connection between the delivery passage 10 and the first blood processing unit 1 when the positive pressure attains a second predetermined pressure value within a predetermined time.

4. A method of ascertaining the presence of a failure in circuit connection, which is executed by a blood purification apparatus designed to separate a blood into a first blood component containing a useful subcomponent, and a second blood component containing a harmful subcomponent by means of a first blood processing unit 1, introduce the blood into the first blood processing unit 1 through a blood introducing passage 8, return the separated first blood component back to a patient's body through a blood return passage 9, and deliver the separated second blood component from the first blood processing unit 1 through a delivery passage 10, which method comprises:
closing a blood return valve 19, disposed in the blood return passage 9, to block a blood circuit including the blood introducing passage 8, the blood return passage 9 and the delivery passage 10;
driving the first pump 4, disposed in the delivery passage 10, in a normal direction, and driving an air pump 70, fluidly connected with the blood introducing passage 8 and also with the blood return passage 9, in a reverse direction to cause air to be discharged from the blood circuit to allow a negative pressure to be developed inside the blood passage and also inside the first blood processing unit 1; and
ascertaining that, when the negative pressure attains a first predetermined pressure value within a predetermined time, the blood circuit and the first blood processing unit 1 are fluidly connected with each other.

5. The method of ascertaining the presence of a failure in circuit connection as claimed in Claim 4, in which the blood purification apparatus is formed as a blood filtering and dialyzing apparatus further comprising a dialysis liquid introducing passage 11 for introducing a dialysis liquid into the first blood processing unit 1, and a second pump 6 disposed in the dialysis liquid introducing passage 11,
wherein the method further comprises:
generating a positive pressure inside the first blood processing unit 1 by driving the second pump 6 in a normal direction to allow air to flow into the first blood processing unit 1; and
ascertaining a fluid connection between the dialysis liquid introducing passage 11 and the first blood processing unit 1 when the positive pressure attains a second predetermined pressure value within a predetermined time.

6. The method of ascertaining the presence of a failure in circuit connection as claimed in Claim 4, in which the blood purification apparatus is formed as a double filtration blood purification apparatus further comprising a second blood processing unit 3, fluidly connected with the delivery passage 10, for separating the second blood component into a high molecular weight subcomponent and a low molecular weight subcomponent; and
wherein the method further comprises:
generating a positive pressure inside the first blood processing unit 1 by driving the first pump 4 in a reverse direction to allow air to flow into the first blood processing unit 1; and
ascertaining a fluid connection between the delivery passage 10 and the first blood processing unit 1 when the positive pressure attains a second predetermined pressure value within a predetermined time.
